# EUROPEAN PATENT SPECIFICATION

(11) **EP 4 457 512 B1**
(45) Date of publication and mention of the grant of the patent: **10.09.2025**
(21) Application number: 22844225.7
(22) Date of filing: 29.12.2022
(51) Int. Cl.: G01N 33/487, B01L 3/00, G01N 33/49, G01N 33/72, G01N 15/00, G01N 15/14

(54) **METHODS FOR IDENTIFYING RECEPTORS AND CELLULAR AVIDITIES**
VERFAHREN ZUR IDENTIFIZIERUNG VON REZEPTOREN UND ZELLAVIDITÄTEN
MÉTHODES D'IDENTIFICATION DE RÉCEPTEURS ET D'AVIDITÉS CELLULAIRES

(30) Priority: 31.12.2021 NL 2030386; 13.05.2022 EP 22173280
(43) Date of publication of application: 06.11.2024
(73) Proprietor: LUMICKS CA Holding B.V., 1105 AG Amsterdam (NL)
(72) Inventor: VAN LOENHOUT, Marinus Theodorus Johannes, 1105 AG AMSTERDAM (NL); GREGG, Trillian Ashley, 1105 AG AMSTERDAM (NL); DAVOLI, Serena Alba, 1105 AG AMSTERDAM (NL); DE GROOT, Mattijs, 1105 AG AMSTERDAM (NL)
(74) Representative: Algemeen Octrooi- en Merkenbureau B.V.
(86) International application number: PCT/EP2022/087999
(87) International publication number: WO 2023/126471

(56) References cited:
- WO-A1-2021/089654
- US-A1- 2021 364 439

## Description

It is understood in the art that immune receptors, TCRs, CARs, and the like, with the highest affinity, when expressed on a cell, may not necessarily give optimal immune responses *in vivo.* Hence, receptor affinity may not correlate with functional performance. Hence, in the art, it is understood that a more important parameter related to and including the specific receptor-antigen interaction between cells is cellular avidity. Cellular avidity, defined as the overall strength of interactions between a diversity of receptor-ligand pairs, involving *e.g*. a specific receptor-ligand interaction, at the cell surface, may provide a parameter that can better predict function. Hence, optimizing and understanding cellular avidity ultimately allows optimizing *in vivo* performance. Hence, there is an interest and need in the art to select *e.g.* TCRs, or the like, which differentiate with regard to cellular avidity.

In the art, identifying or selecting receptors with advantageous and/or different cellular avidities is a highly cumbersome and time-consuming process. Current methods for selecting receptors or the like involving laborious selection criteria utilizing many different screening methods, including cytokine secretion and target cell killing, and also *in vivo* experiments, wherein optionally in the process cellular avidity is determined as well. For many applications, and in particular for personalized medicine, it would be highly advantageous if the process for identifying TCRs, and the like, and its properties, including its cellular avidity, would be highly efficient, in particular from a time perspective, as shortening the time from bench to bedside is ultimately of benefit to patients.

Patent application WO 2021/089654 A1 discloses a known method for measuring a binding force of weakly and strongly bound effector cells from target cells bound to a surface substrate and being pulled by a force away from the substrate. A force ramp is applied to determine cell detachments, record two different avidity curves, and from these data the avidity of the different cells is determined.

Hence, the current inventors now sought to provide for improved means and methods for identifying TCRs and other receptors of interest in correlation to the avidity these receptors bestow on their cells.

### Summary of the invention

The current inventors in seeking for means and methods for identifying TCRs of interest, and the like, and their cellular avidity were aware that in the art, cellular avidity is typically determined by analyzing homogeneous populations of cells carrying a defined TCR, CAR, or the like. For example, utilizing the z-Movi^{®} device as available from LUMICS (see *i.a.* z-Movi^{®}-Brochure_2021.pdf, available from <https://lumicks.com/products/z-Movi^{®}-cell-interaction-studies/#brochure>). Cells of interest carrying different receptors or other modifications, as well as control cells thereof, are typically separately analysed. In working on the problem to provide for improved means and methods for identifying TCRs of interest and their cellular avidity, the current inventors now realized that characterization of a cellular avidity parameter, or cellular avidity score, for a cell of interest having a specific interaction with a target cell (*e.g*. a T cell with a target cell) relies on the measurement of a large number of cell detachment events. This is understood to be *i.a*. because cell detachment, or rupture, from a cell, under force application is a stochastic event, *i.e.* at an applied force there is a certain chance for a cell-cell bond to break. So, even when all attached cells would have an identical bond not all cells would detach at the same applied force. Also, cell-to-cell bond formation (*e.g*. both aspecific binding processes and specific immune synapse formation) is a process taking place in time and goes through different stages and there is a certain distribution of bond formation initiation resulting in not all cells being at the same stage at the same time. Furthermore, in a typical experiment it can be observed that the geometry of a cell-to-cell contact and the surface area over which cells are in contact with each other can vary. Hence, when cellular avidity is determined, this is typically determined by measuring detachment of cells by applying a force and detecting a large number of cells, the collection of detachment events determined in relation to the applied force representing the cellular avidity of a particular cell type. For example, when cellular avidity is determined with an acoustic force, with a force ramp, an avidity curve is provided. To summarize, this means that a cellular avidity score for a cell of interest and a target cell is typically determined by measuring an avidity curve (*e*.*g*. number of cells bound as a function of force) and/or by determining a fraction of bound cells at a certain detachment force (possibly normalized using a control cell pair) for a homogeneous population of target cells and effector cells.

The inventors now realized that advantageously, cellular avidity information on specific cell types and/or specific receptors present on cells of interest can be obtained even when these are comprised in a heterogenous cell population, provided that such cells of interest are represented as a cell clone population. Cellular avidity information can in accordance with the invention be obtained by contacting the heterogenous population with target cells, applying a force and collecting one or more fractions, after which subsequently cell clones are identified and quantified in the cell fractions. Fractions to be obtained include detached cells and/or attached cells, and/or a fraction sampled from the input cell population. By determining and quantifying e.g. the sequences of the TCRs or CARs from single cells in fraction(s), a cellular avidity score can be assigned to one or more TCRs or CARs, or the like, comprised in the population (even in the case when such TCR or CAR sequences were previously unknown). Hence, valuable information concerning the cellular avidity of TCR receptors, or the like, can be obtained and subsequently ranked, in a single measurement without having to resort to isolating cell clones and analysis thereof separately.

For example, when starting out with a heterogeneous cell population comprising T cells with two known CAR receptors present therein, wherein one has a low cellular avidity score and the other has a high cellular avidity score for defined target cells, for example as determined with a z-Movi^{®} device or the like. When this heterogenous cell population is allowed to interact with target cells, and subsequently an increasing force is applied to the cells, fractions of cells that detach from the target cells at the lower ranges of the applied increasing force will have the cells with a low cellular avidity relatively overrepresented, whereas the cells with a high avidity score will be relatively overrepresented in fractions at the higher ranges of the applied increasing forces. The (relative) cellular avidity can be determined for CAR receptors or the like in a heterogenous cell population by isolating fractions and assessing the distribution of unique cell clones present across the fractions (i.a.. by assessing for a unique clone of relevance the number of cells in fractions representing such unique clone).

Cells representative of a cell clone population can easily be identified *e.g*. as cell clones can be genetically distinguishable (*e.g*. via a nucleic acid sequence tags). Advantageously, one can also simply determine and quantify receptor sequences (or at least the variable region thereof) of a cell clone present in fractions instead (*e.g*. by determining copy number, *e.g*. per cell), the unique sequences of receptors identifying cell clones being of prime interest. As said, there is thus no need to resort to isolating individual cells, cloning of TCR receptors or the like, and determining cellular avidity scores for separate receptors. The more cells detected in fraction(s), such as bound and/or unbound fractions (*i.e.* attached and/or detached), the more confidence can be obtained with regard to cellular avidity scores, and/or relative cellular avidity. This may also depend on differences between cellular avidity. Meaning, if the cellular avidity difference between two cell clones is small, in order to correctly rank these according to their respective cellular avidities, more cells from the cell clone populations need to be identified and quantified in fractions as compared with a scenario wherein the cellular avidity difference may be relatively large.

Hence, the current inventors now provide for new means and methods to, highly advantageously, determine cellular avidity for individual TCRs or CARs or the like from a heterogenous cell population, provided off course that cell clone populations *(i.e.* multiple cells with at least the same receptor) are present in the heterogenous cell population. Using the advantageous means and methods in accordance with the invention, cellular avidity can now be easily determined from heterogenous cell populations.

### Figures

FIG 1A: averaged avidity curves obtained on homogeneous populations of three different cell clones of primary T cells. One untransduced (UNT) and two transduced with specific TCR sequences (TCR1 and TCR2).
FIG 1B: zoom-in on the avidity curves of FIG 1A.
FIG 2: representative individual avidity curves from the dataset used in FIG 1 showing raw cell counts per run.
FIG 3: simulated experiment where cells from different runs from the dataset of FIG 1 are mixed together *in-silico.*

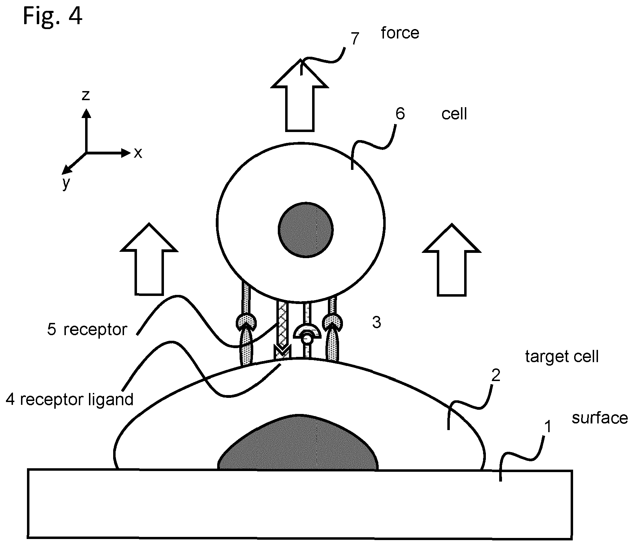

FIG 4: Schematic showing target cells and cells of interest with a receptor and cellular avidity. Target cells (2) are provided on a surface (1), which as depicted is in this case a flat surface. The target cell expresses ligands and receptors, likewise the cell to be targeting the target expresses ligands and receptors as well (3). A specific ligand-receptor interaction (4 and 5) can be the driving force for the forming of a cell-cell bond with multiple ligand-receptor interactions combined resulting in strong cell-cell binding. To rupture this cell-cell bond, a force (7) is exerted on the cell (6) away from the target cell (2), which can be in the z-axis direction *e.g*. when the flat surface is defined as being in the x-y plane. Alternatively, this can also be in the x- or y-axis direction. When the cell-cell bond is ruptured, the cell moves away from the cell surface and this event can be detected and/or detached cells can be collected and quantified and/or further analyzed.

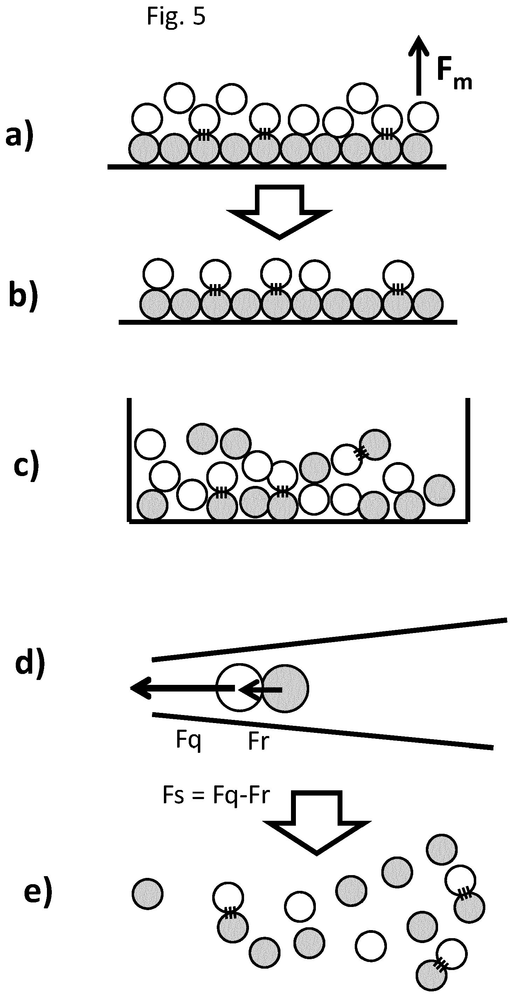

FIG.5: In cellular avidity methods with cells attached to a surface (depicted as grey cells), cells (depicted as white cells) are interacted with the attached cells to bind therewith, *e.g*. utilizing target cells expressing an antigen and cells carrying a receptor such as effector cells with a CAR against the antigen, as depicted in a). After a defined incubation, a force, Fₘ applied away from the attached cells, *e.g*. target cells. This results in effector cells moving away therefrom, either because they did not bind to the cells attached to the surface or because the binding strength was not strong enough, *e.g*. when aspecifically bound. Cells that remain were sufficiently strongly bound to the attached cells as depicted in b). Such a scenario is *e.g*. employed in the examples as described herein. Cells that formed a synapse may substantially remain (indicated with hashes). Alternatively, a cellular avidity method can be performed which does not require cells attached to a surface. In this scenario, *e.g*. target cells (depicted as white cells) are provided and cells of interest, *e.g*. effector cells (depicted as grey cells), and these are incubated for a defined period. After said incubation, cells are resuspended and a differential force, Fₙ, is applied. Such an applied force may be larger than typically used when cells are attached (Fₘ). This force may break cell-cell bonds, preferably aspecific cell-cell bonds as these bonds may be less strong when compared with specific cell-cell bonds such as *e.g.* synapse bonds, thereby resulting in *e.g.* substantially specific cell-cell bonds. The cell suspension may be subsequently analysed with regard to singlets (either white or grey cells) and doublets (grey cell bound with white cell), which numbers may be used, *e.g*. to provide a cellular avidity score. In case of a heterogeneous population of cells comprising a plurality of cell clone populations carrying a receptor, such singlets and doublets can be separated to provide cell fractions and cell clones can be identified in said fractions, in accordance with the invention. Of course, one may also combine applying a differential force (shown in d), after a force has been applied, *e.g*. away from attached cells (in the scenario obtained as shown in b), or one may apply a differential force after incubation on attached cells (in the scenario as obtained in a). Cellular avidity scores for cell clones identified may be determined by determining for a cell clone at least the number of grey cells and white cells bound to each other (doublets). It is also possible to determine cellular avidity scores based on the number of cells representing identified cell clones based on the number of grey cells bound to white cells, *e.g*. effector cells bound to target cells, that formed a specific bond such as a synapse (doublets with hashes). Cellular avidity scores can be calculated taking into account initial amount of cells provided and/or single cells obtained in the method. For example, by calculating of identified cell clones the number of cells that remained bound to target cells after exerting the force, the ratio or percentage, relative to the number of cells of identified cell clones initially provided.

### Detailed description

In a first embodiment, a method is provided for determining the cellular avidity of a plurality of cell clones with a receptor, capable of binding target cells, comprised in a heterogenous population of cells by:
a) providing a heterogenous population of cells comprising a plurality of cell clone populations carrying a receptor;
b) providing target cells attached to a surface;
c) contacting the heterogenous population of cells with the target cells;
d) applying a force on the heterogenous population of cells away from the target cells;
e) collecting detached cells to provide for at least one cell fraction;
f) identifying cell clones comprised in the fraction(s) provided in step e), and determining the number of cells representing identified cell clones in the fraction;
g) assigning cellular avidity scores to identified cell clones based on the determined numbers of step f).

Hence, the method in accordance with the invention is for determining the cellular avidity of a plurality of cell clones with a receptor. These cell clones are to bind to the target cells, *i.e.* via a receptor, and the means and methods in accordance with the invention are to determine the capability, which may be more or less as can be assessed with the cellular avidity score, of these cell clones with a receptor to bind to the target cells, Hence, it is not a requirement that all cells comprised in the heterogeneous cell population are capable to the same extent with regard to binding to the target cells and some cell clones may not bind at all. It is understood that "cellular avidity" as used throughout herein comprises the overall strength of interactions occurring in a cell-to-cell contact, involving a diversity of molecules at the surfaces of the cells that interact (see *e.g*. Figure 4). Such interactions may include a diversity of receptor-ligand pairs, among which *e.g*. a specific receptor-ligand interaction, occurring at the membrane surface of a cell. For example, when a T cell receptor triggers the formation of an immune synapse by recognizing an antigen presented by an MHC molecule at an antigen presenting cell, the synapse formation involves such multitude of interactions, as also other membrane bound molecules are involved in the interactions (such as integrins and the like). Hence, "cellular avidity" may not be restricted to the interaction of *e.g*. the alpha and beta chain of the TCR and the antigen presented by MHC, but rather involves a multitude of interactions working jointly forming a strong bond between *e.g*. cells. It may also involve active signalling and processes internal to the cells such as *e.g*. during immune synapse formation. It is understood that the cellular avidity of a cell of a certain type is defined relative to its target cell and conditions tested. Of course, it is not a requirement to determine the cellular avidity of a plurality of cell clones, one may also determine the cellular avidity of a single cell clone from the plurality. Hence, in another embodiment, means and methods are provided for determining the cellular avidity of at least one cell clone with a receptor, capable of binding target cells, comprised in a heterogenous population of cells by:
a) providing a heterogenous population of cells comprising a plurality of cell clone populations carrying a receptor;
b) providing target cells attached to a surface;
c) contacting the heterogenous population of cells with the target cells;
d) applying a force on the heterogenous population of cells away from the target cells;
e) collecting detached cells to provide for at least one cell fraction;
f) identifying at least one cell clone comprised in the fraction(s) provided in step e), and determining the number of cells representing the at least one identified cell clone in the fraction;
g) assigning cellular avidity scores to identified cell clone(s) based on the determined numbers of step f).

Hence, the current invention relates to methods for determining cellular avidity. In particular, methods of the invention relate to means and methods in which a plurality of cell clone populations with a receptor comprised in a heterogeneous population is provided. In any of the methods as described herein, it may be contemplated to determine the cellular avidity score only of one cell clone. However, it is preferred and highly advantageous to determine the cellular avidity of more cell clones, as it may also advantageously allow one to rank cell clones.

It is understood that a cell clone with a receptor in accordance with the invention means a cell which expresses a defined receptor sequence, *e.g*. a defined CAR sequence or defined alpha and beta chains of a TCR, or the like. Of the cell clones a plurality is provided, which is understood to comprise a plurality of cell receptors, *i.e.* receptors that are different with regard to their sequence. Of cell clones with a receptor, a population of cells is provided as well. It is not required that all cell clones are provided as a population of more than one cell and some cell clones may be present in low cell numbers and/or identified only once. It is understood that in a cell clone population the cells comprised in that population carry the same receptor. Hence, phrased differently, a heterogeneous population of cells accordingly is to comprise a plurality of different receptors, wherein a receptor can be presented by a cell clone population of cells, *i.e.* multiple cells carrying the same receptor. For example, a library of CAR-T cells may be prepared from a lentiviral vector library encoding 100 different CAR sequences. With this vector library, 100,000 cells may be transduced. In accordance with the invention, transduced cells carrying the same CAR sequence may be referred to as a cell clone population, which population size is on average 1000 cells, and the plurality of cell clones may be referred to as 100. Of course, the transduced cells may be grown or expanded to 1000,000 cells, which increases the average size of a population to 10,000, whereas the plurality of cell clones, or cells carrying a unique receptor (construct) remains 100. Likewise, the same applies to *e.g.* primary cell samples, in which *e.g.* clonal expansion may have occurred *e.g*. in case of T cells or the like.

Of cell clone populations, subpopulations may also be further identified, if of interest. For example, in a scenario wherein *e.g*. different donors would be used, cell clone subpopulations for each donor may be identified. Or, when for example different types of cells would be used. Different donors and/or different types of cells may be labelled differently and/or identified *e.g*. based on intrinsic genetic differences between the cell types or based on specific barcodes artificially introduced in the cell. This can provide in addition to cellular avidity *per se,* a further complexity to the analysis by which cellular avidities can be determined and compared across cell receptors, and/or across different cell types and/or different donors. Moreover, it may be envisioned that (in part) the heterogeneity of the cell clones may not necessarily involve the receptor *per se,* but may also involve cellular protein factors or the like having an effect thereon, in such a scenario cell clones may be identified (in part) by the (sequence) variation in such factors, *e.g*. co-factors that may be involved in processing and/or transport or the like that may have an effect on cell receptor function.

In any case, it is a prime object in accordance with the current invention that the methods of the invention are in particular useful for the analysis of a heterogenous cell population carrying a plurality of cell clones, *e.g*. representing 10 or more receptors, and that by applying the methods of the invention, without requiring to know the exact composition of the heterogenous cell population and/or receptor sequences and function, cell clones can be identified therefrom and cellular avidity for cell clone receptor sequences can be determined. In one embodiment, the heterogeneous cell population preferably is composed of cell clone populations whereof the exact receptor sequences and/or the number of cells therewith as present in the cell clone populations therein is not known *a priori.* In another embodiment, the heterogeneous cell population is not composed by combining cell clone populations. Of course, one can always prepare or provide cell clone populations separately and combine these to provide for a heterogeneous population, but the means and methods allow one to not needing to provide separate cell populations with defined receptors in order to be able to identify receptors and their corresponding cellular avidity.

In another embodiment, the number of cell clone populations, *i.e.* the plurality, represents at least 2 or more, 3 or more, more preferably at least 5 or more, most preferably 10 or more cell clones, *i.e.* cells carrying different receptors. The latter may be assumed when *e.g*. primary cells are provided as a heterogenous cell population, and is off course well controlled when *e.g.* variants of CAR receptors or libraries thereof or the like are used to prepare a transduced or transfected heterogenous cell population. In yet another embodiment, the number of identified cell clones is at least 1, at least 2, at least 3, at least 4, or more. The number of (identified) cell clones largely depends on the complexity of the heterogeneous cell population and may not be restricted, as long as sufficient cell numbers representing cell clones can be identified to allow for assessment of cellular avidity, *i.e.* determine a cellular avidity score, such may be contemplated.

Hence, accordingly, in step a) of the invention, provided is a heterogenous population of cells comprising a plurality of cell clone populations carrying a receptor. Furthermore is provided in step b) target cells attached to a surface. It is understood that in accordance with the invention target cells and cell clones with a receptor relate to two different cells which are to interact specifically with each other, *e.g*. one cell expressing a ligand on its surface whereas the other cell expresses a receptor for that ligand. The term ligand and receptor in this sense and in accordance with the invention referring to defining their inter-relationship, and the cells of which a heterogeneous cell population is provided are to have variation in the protein (receptor) presented on its cell surface. The term receptor may not be construed to be limiting in any way and is understood to mean a protein presented at the cell surface which can (specifically) interact with another protein (ligand) presented at another cell. The terms ligand and receptor are used to indicate a complementarity which is important for specific recognition between cells without restrictions on the complementary molecules that can be contemplated. Of particular interest and as further described below, in accordance with the invention, the cells with a receptor can be T cells or the like, which are to specifically bind a target cell *e.g*. a cancer cell or other antigen presenting cell presenting a defined antigen on its surface.

The target cells are provided attached on a surface. Providing the target cells attached to a surface is well known in the art. For example, a glass or plastic surface may be utilized to attach cells thereto. A surface material may be preferred which allows for detection of attachment of cells to target cells and/or detachment of cells from the target cells. Such a surface material for instance also may allow for microscopy methods (*e.g*. by being a transparent material). In order to attach cells to the surface, the surface may be pre-treated with a coating such as a polypeptide. Suitable polypeptides include *e.g*. poly-L-lysine or the like. Suitable polypeptides for attachment of target cells that may be contemplated and are known in the art include for example fibronectin, poly-L-lysine, poly-D-lysine, poly-L-ornithine, laminin, collagen, fibronectin, fibrinogen, vitronectin, or osteopontin. In any case, a suitable polypeptide or other suitable coating may be selected such that the target cells that are attached to the surface allow for the target cells to remain attached to the surface when applying a force on the heterogeneous population of cells. In other words, when a force is applied on these cells which allows for detachment of (some) of these cells from the target cells, the target cells are to substantially remain attached to the surface.

In the next step, step c), the heterogenous population of cells is contacted with the target cells. Hence, in this step, the heterogenous population of cells are introduced on the target cells, *e.g*. by layering the cells thereon. It is understood that this step of contacting is well controlled. For example, as shown in the example section, the step of contacting may be a defined period of 5 minutes. Of course, the step of contacting may be shorter or longer, for example in the range of 1 minute to 30 minutes, or from 2 minutes to 15 minutes. In any case, for a defined period, the cells are to be in allowed to remain in each other's vicinity such that the receptor and ligand may interact and form a bond.

After the incubation step, a force in step d) is applied on cells that interacted with target cells, away from the target cells. It is understood that in this step, the force applied may be perpendicular (in the direction of z-axis) to the surface (x,y) to which the target cells are attached, for example when a centrifugal force or acoustic force is applied (see Figure 5). The force may also be lateral (x-axis or y-axis), for example when a shear force is applied. In any case, the force is applied and is controlled such that a defined force is exerted on the cells that interact with the target cells. It is understood that the force that is exerted on the cells attached to the target cells is to be substantially equal, such can be achieved *e.g*. when using a flat surface as depicted in Figure 4. Other suitable surface shapes may be used (*e.g*. a tube with exerted concentrical force or laminar flow force in the direction of the length of the tube), as long as the force exerted can be substantially equal at a defined surface area, such a surface shape may be contemplated. The force required to move a cell away from the target cell preferably can be detected, *e.g*. via microscopy or other means, to which may be referred to as a cell detachment event. This way, cell detachment events can be monitored and counted. As long as the force applied at the moment of the detachment event is known, detachment events may be correlated to forces.

Next, in step e) the detached cells are collected to provide for at least one cell fraction. It is understood that the cells that are detached may preferably all captured. These may be collected in one fraction, but may also entail the collection of multiple fractions, for example, when a force ramp would be applied (see below). Subsequently, the cell clones comprised in the fraction(s) provided in step e) are identified, and the number of cells representing identified cell clones in the fraction is determined in step f). Based on the determined number of an identified cell clone, in step g) cellular avidity scores are assigned to identified cell clones based on the determined numbers of step f).

As shown in the example section, the determined number of cells representing an identified cell clone in the detached cells, combined with either knowing at least the number of cells representing the cell clone in the heterogeneous cell population and/or the number of cells representing the identified cell clone in the attached cells, allows one to determine a cellular avidity score. Of course, it may not necessarily be required to have more than one or all of these numbers, when *e.g*. it is known or expected a provided heterogeneous cell population has normal distribution of cell clone populations, *e.g*. when substantially the same amounts of viral vectors are used in generating a library. In such scenarios, the lower the number of an identified cell clone in the detached fraction, the higher the (expected) cellular avidity may be.

In any case, as long as *e.g*. the number of cells representing identified cell clones can be determined, or a representative part thereof such that the percentage or abundance of identified cell clones can be determined relative to the heterogeneous cell population, this may provide sufficient information to determine cellular avidity. Of course, one could also instead of, or in addition to, collecting the detached cells, collect cells that remain attached to the target cells at the end of the applied force in step e).

In another embodiment, a method is provided for determining the cellular avidity of a plurality of cell clones with a receptor, capable of binding target cells, comprised in a heterogenous population of cells by:
a) providing a heterogenous population of cells comprising a plurality of cell clone populations carrying a receptor;
b) providing target cells attached to a surface;
c) contacting the heterogenous population of cells with the target cells;
d) applying a force on the heterogenous population of cells away from the target cells;
e) collecting cells that remain attached to the target cells to provide for at least one cell fraction;
f) identifying cell clones comprised in the fraction(s) provided in step e), and determining the number of cells of identified cell clones in the fraction ;
g) assigning cellular avidity scores to identified cell clones based on the determined numbers of step f).

Highly preferably, in the methods above, wherein either at least the cells that remain attached to the target cells or the detached cells are collected, the composition of the heterogenous population of cells provided is determined, *i.e.* at least of identified cell clones the number is determined. Alternatively, the heterogenous cell population is defined *a priori,* or known or assumed to be unbiased with regard to its clonal composition.

Hence, in a further embodiment, of the heterogenous population of cells provided in step a) a fraction is collected of the provided starting population in step a) which represents a further fraction provided in step e); and/or wherein the heterogenous population of cells provided in step a) is defined with regard to cell clone populations comprised therein. This way, based on the relative increase or decrease of an identified cell clone number (or abundance, *e.g*. fraction or percentage relative to total, part of total, or relative to the identified cell clones), when comparing detached or attached cells as compared with the heterogeneous population, cellular avidity can be determined of an identified cell clone and its receptor sequence. It may be preferable to determine the number or abundance of cells representing identified cell clones in the sample population only after such clones have been identified in the fraction of cells that remain attached. This may allow the sequencing method to be targeted specifically at those cell clones of interest (that have a higher likelihood of being high cellular avidity cell clones and/or cell clones that specifically recognize and bind to the target cells). This way, determining of the number or abundance of cells representing identified cell clones in the starting population may be more efficient.

In another embodiment, a method is provided for determining the cellular avidity of a plurality of cell clones with a receptor, capable of binding target cells, comprised in a heterogenous population of cells by:
a) providing a heterogenous population of cells comprising a plurality of cell clone populations carrying a receptor;
b) providing target cells attached to a surface;
c) contacting the heterogenous population of cells with the target cells;
d) applying a force on the heterogenous population of cells away from the target cells;
e) collecting detached cells to provide for at least one cell fraction; and collecting cells that remain attached to the target cells to provide for a further cell fraction;
f) identifying cell clones comprised in the fractions provided in step e), and determining the number of cells representing identified cell clones in the fractions;
g) assigning cellular avidity scores to identified cell clones based on the determined numbers of step f).

In any case, it is important to know or determine identified cell clone numbers of at least two of 1) the heterogeneous cell population, 2) detached cells, and 3) attached cells. It may also be contemplated to rank identified cell clones based on identified cell clone numbers or abundancy from two detached fractions, which have detached at different forces. This way, a cellular avidity score can be calculated which allows for the ranking of identified cell clones and their receptor sequences. As it may be highly preferred to identify cell clones using sequencing methods, *e.g*. by sequencing variable regions of the receptors, it may be advantageous to first identify in attached cells identified cell clones with relatively high numbers, as these may have a higher chance of representing cell clones with high cellular avidity and/or which specifically recognize and bind to target cells. Hence, it may not necessarily be required to sequence all fractions at the same time, but one may *e.g.* first perform an analysis of an attached cell fraction first, and subsequently perform a selective sequence analysis focused on identified cell clones.

Instead of providing live target cells attached on a surface, a functionalized wall may also be provided which provides the heterogeneous population of cells with a specific surface to attach to. In another embodiment, instead of providing target cells attached on a surface, a functionalized wall can be provided which provides cells of interest, *e.g*. a heterogeneous cell population as provided herein, with a suitable surface to attach to in the means and methods in accordance with the invention as described herein throughout. A functionalized wall in accordance with the invention presents ligands/receptors in a similar fashion as they are presented on a cell surface, *e.g*. in a lipid bilayer, or the like. A functionalized wall thus preferably is functionally equivalent to target cells attached to a surface, and mimics target cells attached to a surface. Hence, in another embodiment, in accordance with the invention, a method is provided for determining the cellular avidity of a plurality of cell clones with a receptor, capable of binding a target on cells, comprised in a heterogenous population of cells by:
a) providing a heterogenous population of cells comprising a plurality of cell clone populations carrying a receptor;
b) providing functionalized wall presenting a target;
c) contacting the heterogenous population of cells with the functionalized wall;
d) applying a force on the heterogenous population of cells away from the functionalized wall;
e) collecting detached cells to provide for at least one cell fraction and/or cells that remain attached to the target to provide for another cell fraction;
f) identifying cell clones comprised in the fraction(s) provided in step e), and determining the number of identified cell clones in the fraction ;
g) assigning cellular avidity scores to identified cell clones based on the determined numbers of step f).

In another embodiment, in the heterogeneous population of cells provided in step a) a defined portion of control cells and/or reference cells is included in addition. A portion of control cells and/or reference cells includes *e.g*. a defined percentage of the total number of the heterogeneous population of cells to not carry a receptor (or not specific to the target cells, and/or with a reference receptor specific to the target cells). The cells not carrying a receptor serving as a control for not having a low or undesired cellular avidity, whereas the cells with a reference receptor serve to allow to assess the cellular avidity of to be newly found receptors relative thereto. It may not be necessary to know the exact portion or defined percentages of these cells *a priori,* as like for the other cells in the heterogeneous cell population, cellular avidity can be determined without that information. All that is required, is being able to identify the control cells and/or reference cells. This may also advantageously allow for controlling the process, as for the process to be acceptable detected cellular avidity parameters may be set for the control cells and/or reference cells. For example, in case these cells are optionally differently labelled, this can be easily determined, without yet needing to identify cell clones and their numbers, and when the cellular avidity or avidities are acceptable, one can commence with the identifying step.

As said, it may be preferred to identify a cell clone by determining the sequence of a receptor, or at least the variable region thereof and also quantifying the number of cells representing cell clones present based on the sequence (*e.g*. by using nucleic acid sequence quantification methods). A multitude of means and methods suitable therefor are known in the art (Pai, J. A., & Satpathy, A. T. (2021) Nature Methods, 18(8), 881-892). Engineered receptors such as used in CAR T cells generally use a single chain antibody sequence, therefor not requiring to identify receptor chain sequence pairs as is the case for TCRα and TCRβ chains.

Identification of cell clones and numbers from an avidity fraction of a library of (single-chain) CAR-T cells can be performed as follows. The different clonal populations in the CAR-T screening library (preferably with 100-10000 cells per clone) will have uniquely modified receptors and the aim of cellular avidity fractionation and sequencing is to determine the cellular avidity of identified cell clones. This can be achieved by looking at the ratios of genomic copies of a fragment that includes the variable receptor region. Since each cell, in most cases, is expected to contain a single integration of the receptor sequence this provides an accurate representation of the clonal numbers in a fraction. Cells are collected in separate fractions as cell suspension or may also be recovered as cell lysate (in the case of the attached cells a lysis solution may be used to obtain the material directly from the sorting device). A genomic region including the variable receptor region is subsequently amplified using a pair or a pool of sequence specific primers. A unique barcode, also referred to as unique molecular identifier UMI, may be present in the primers and these will be included in the first 1 or 2 rounds of PCR amplification. Primers with these UMIs may then be removed and further cycles of amplification are performed. After sequencing, counting of the number of UMIs for a unique receptor sequence allows for an accurate estimation of the cell numbers for a clone without artifacts due to PCR bias.

For combinations of receptor pairs, such as an alpha and beta chains, combinatorial pairing of receptor chains and estimation of clonal fractions in a population of clonal T cells may be performed. In a population of T cells where there are expected to be multiple cell clones and each clonotype is present in multiple cells *e.g.* 1000 different clones with 10 to 100 cells in each clone, combinatorial analysis of sequencing may be used to infer chain pairing. An avidity fraction of cells is split into multiple subfractions *e.g*. 384 separate fractions and from each of these subfractions an indexed sequencing library is prepared; these can be either genomic or transcriptomic sequencing libraries made using receptor specific primers. After sequencing, the pairing of receptor chains may be determined by statistical analysis of the co-occurrence of receptor pairs in subfractions. The abundance of individual clones in a cellular avidity sorted fraction may be determined by counting the number of subfractions in which it is present and comparing that to the Poisson distribution.

Furthermore, single-cell sequencing for pairing of receptor chains may be performed. Single-cell library preparation methods are generally more expensive and complex but offer several advantages, including a) simple counting of clonal fractions as cells are individually barcoded, and b) receptor chain pairing as separate chains form the same cell can be identified. In one implementation single-cell libraries may be prepared by dispensing individual cells from an avidity fraction into well plates by FACS or other single-cell dispensing methods and preparing full-length RNA sequencing libraries using established protocols *e.g*. Smart-seq3. These libraries can be sequenced directly or further enriched by targeted amplification using specific PCR primers for receptor sequences or other genes of interest. Similar libraries may also be prepared using droplet based single-cell RNA sequencing methods, *e.g*. 10X genomics or InDrop. After sequencing receptor pairs, clonal cell numbers and other genes of interest can be bioinformatically quantified using the single-cell barcodes and UMIs incorporated during the library preparation.

Although it may be preferred to determine the sequences of the receptor sequences, or at least the variable region thereof (*i.e. e.g.* CDR sequences or other regions of the receptor that have been varied, *e.g*. in case of designed receptors such as CAR-T receptors), one may also detect and quantify cell clones by including *e.g.* a genetic label in cells. Hence, such a label may serve to identify a receptor instead of sequencing it. Such may be advantageous *e.g*. in a scenario wherein vector libraries are generated.

In any case, in the methods in accordance with the invention preferably, the number of cells representing a cell clone is determined by quantifying nucleic acid sequences representing a unique cell clone. Alternatively, other means and methods aimed at identifying and quantifying the unique amino acid sequence as encoded by the receptors in cell clones may be contemplated. The numbers obtained can be used to calculate abundancy (fraction or percentage comprised in the obtained fractions provided in step e), and the numbers and abundancy can be used to calculate the cellular avidity scores. For example, in order to determine only the relative cellular avidity, it may be sufficient to determine only the relative abundance of identified cell clones in the heterogeneous cell population and in the detached or attached cell fraction. One can calculate the relative cellular avidity by dividing the relative abundance of the sorted attached fraction (or alternatively 1 minus the relative abundance of the sorted detached fraction) with the relative abundance in the heterogeneous cell population. The relative cellular avidity also indicates the relative enrichment. If one is interested in the absolute cellular avidity, with respect to the specific experimental conditions, then it is important to determine the abundance of these cell types in the cell sample in relation to the total number of cells that are allowed to incubate onto the functionalized wall surface or target cells (*i.e.* in relation to the total number of cells that are effectively sorted in detached and attached fractions by applying the force). Alternatively, one can also deduce these numbers based on analysing both detached and attached fractions. This way, the percentage of the cells that remains bound represents absolute cellular avidity. Such an absolute cellular avidity is of course dependent on the testing conditions, and when the same testing conditions are used, this allows for comparing different cell clones from independent experiments and rank these accordingly. Hence, in one embodiment, the cellular avidity score is either a relative cellular avidity score or an absolute cellular avidity score. As these numbers are calculated by determining a ratio, these may be presented as percentages. Hence, in another embodiment, for an identified cell clone, the percentage of cells that remained bound relative to the initial cell clone population (*i.e.* 100%) comprised in the heterogenous population is calculated as a cellular avidity score.

As said, determining the cellular avidity allows one to rank identified cell clones. In particular in the scenario wherein multiple different analyses are performed it may be preferred to determine the absolute cellular avidity. For example, an experiment may be conducted multiple times and/or with multiple different samples and/or on multiple different devices. By determining the absolute cellular avidity, cellular avidity scores differentiating between identified cell clones may be advantageously compared though not necessarily being determined in the same analysis. Of course, if one ensures that for each experiment, at least approximately, the same number of cells are contacted and exposed to force, then one can still rank cells from independent experiments even if one does not know how many cells are sorted in each experiment.

In a further embodiment, the applied force in the means and methods of the invention is a force ramp, preferably a linear force ramp. It is understood that the force that is to be applied can be a constant force applied for a defined period. The forces applied may be in various forms as a function of time. Preferably however, the applied force is an increasing force, that is, after the incubation step, an increasing force is applied for a defined period until a defined end force is reached. Increasing the force is preferably done as a linear force ramp, but other ways to increase the force over time may also be used (*e.g*. exponential loading where the force is doubled over a certain small time period and keeps doubling until an end force is reached. For example, as shown in the example section, in about 150 seconds, a defined end force is reached of 1000 pN. An increasing force may be referred to as a force ramp, which may preferably be a linear force ramp, but other ways of increasing the force may be contemplated. As said, detached cells can be collected in a fraction. It may also be opted to collect cells in different fractions, *e.g*. each fraction representing a defined period in which the force (ramp) was applied. By collecting different detached fractions, one may *e.g.* construct a cellular avidity curve.

Any suitable force application method may be contemplated in accordance with the invention. Increasing the force can be well controlled with acceleration based methods of applying force such as centrifugation, with shear flow and with acoustic force, which are all suitable means to be used in the methods in accordance with the invention but any other means of controllably causing a force on the cells attached to the target cells, thereby forcing them away from the target cells or the functionalized wall surface, may be contemplated. Accordingly, in a further embodiment, the force that is applied is selected from is an acoustic force, a shear flow force or a centrifugal force.

It may be advantageous to apply shear flow and/or centrifugal flow in scenarios wherein a large number of cells are to be tested. Acoustic force, though very well suitable in the means and methods of the invention, allows for the processing of about 100 - 10,000 cells, *e.g*. utilizing a device like the z-Movi^{®} device as available from Lumicks. This is because the surface area at which the acoustic force may be well controlled can be limited. However, using centrifugal force and/or shear flow, the forces exerted on the cells that attach to the target cell layer can be more easily controlled over much larger areas allowing for the sorting of millions to billions of cells.

With regard to the cellular avidity, and determining a cellular avidity score, it is understood that this is to express the strength of binding of cells of interest, *i.e.* cells carrying a receptor (or control cells thereof) to the target cells. It is understood that where we refer to specific forces applied to cells this may refer to average forces, *e.g*. such forces may not be fully homogeneous, for example over the contact surface as may be the case with acoustic forces and shear-flow forces (see *e.g*. Nguyen, A., Brandt, M., Muenker, T. M., & Betz, T. (2021). Lab on a Chip, 21(10), 1929-1947) for a description of force inhomogeneities in acoustic force application).

Also for shear-flow forces, the forces may not be fully homogeneous, for example since the flow speed near the side walls of a flow channel (*e.g*. with a rectangular cross section) may be lower than in the center of the flow cell (due to the no-slip boundary condition). By choosing a cross section with a high aspect ratio (low and wide) these flow effects may be minimized such that only a few percent of the cells experience a substantially smaller force than the cells in the center of the flow cell. Other methods to mitigate such effects and to specifically select cells that have experienced similar forces may include using flow cell geometries with multiple fluid inlets and/or outlets such that the properties of laminar flow can be used to ensure cells of interest only land in regions of homogeneous force and/or are only selected from regions of homogeneous force. In one example, by using three channel inlets side by side one can use the side channels as sheath flow channels to focus cells of interest inserted into the central channel inlet towards the center of the interaction region where the acoustic and/or shear force may be substantially homogeneous. The sheath flow fluid may be the same buffer fluid as is used for the sample cells but then free of sample cells. By increasing the flow speed through the sheath flow channels the cells are more focused and confined to the center of the channel while by reducing the sheath flow speed the cells are allowed to spread out more. Similarly, on the collection side, flows in three side-by-side collection channels may be controlled to possibly discard cells flowing close to the channel boundaries and only collecting cells from the center of the channel. By controlling the relative flow speeds of such side channels and the center channel asymmetrically the location of the effective interaction region of the sorting device can be further controlled and cells that have underwent defined forces can be selected and/or detected.

Further means to enable collection of cells from a specific interaction region (and therefore collection of cells that experienced a defined force) include means and methods wherein cells of interest may be provided with a photoactivatable label which may be subsequently activated by illumination with light of a suitable wavelength only in a well-defined interaction region of the device (*e.g*. near the center of a flow channel or in a center region under an (acoustic) force transducer) to photoactivate and/or switch the dye. Subsequently, the cells can be sorted for example using fluorescence activated cell sorting (FACS) and only those cells which are activated are further used according to the methods described herein thereby obtaining the cells on which defined forces have been exerted. This may for example be highly useful for collecting cells that remained attached to the target cells. For example, the target cells and cells bound thereto may be trypsinized thereby obtaining both the target cells and cells that remained bound thereto in a suspension. Alternatively, attached cells can also be simply collected with physical means (*e.g.* scraping) from the area of interest, *i.e.* the surface area with a well-defined nominal force.

For centrifuge forces, it is easier to ensure that the force applied is homogeneous across the whole interaction region, since such a force does not depend strongly on a location on a surface with respect to a force transducer and/or the wall of a flow channel or sample holder.

Accordingly, in connection to the subject matter disclosed herein, means and methods exist which allow one to exert forces on cells attached to a surface and collect the cells, detached and/or attached cells, on which defined forces have been exerted.

It is understood that with regard to the force exerted, the exact forces experienced by cells may also depend on cell size and or other cell properties such as density and compressibility. The force may be a nominal force and not the true force experienced by the cells. *E.g*. it may be hard to precisely predict the average cell size, density, compressibility, etc. of the cells and the force may have been calculated based on theory alone or may have been calibrated using test particles with specific (preferably known) properties (see *e.g*. Kamsma, D., Creyghton, R., Sitters, G., Wuite, G. J. L., & Peterman, E. J. G. (2016). Tuning the Music: Acoustic Force Spectroscopy (AFS) 2.0. Methods, 105, 26-33). The force may be such a calculated or calibrated force expressed with units of N (*e.g*. pN), but it may also be expressed without calibration as the input power (Vpp) applied to a piezo element (see Sitters, G., Kamsma, D., Thalhammer, G., Ritsch-Marte, M., Peterman, E. J. G., & Wuite, G. J. L. (2014). Acoustic force spectroscopy. Nature Methods, 12(1), 47-50), as angular velocity squared (*ω²*) in the case of centrifugal force application or as flow speed *v* and or as shear stress (Pa) in applications using shear forces. As long as the forces exerted by the devices, *e.g*. shear force, acoustic force, or centrifugal forces, but not limited thereto, can be varied and controlled and reproduced in such devices such devices are suitable for the means and methods in accordance with the invention.

In another embodiment, the target cells are attached to a glass surface, preferably a glass surface in a chip or the like (such as described *i.a*. in Fernández de Larrea, C. et al. (2020). Blood Cancer Discovery, 1(2), 146-154) with target cells attached to its surface. Such a chip is also described in US10941437B2 and WO2018083193. The target cells that are attached to the surface, preferably are attached as a monolayer. The monolayer preferably is at high confluency. The subsequent cells that are to interact with the target cells are preferably provided in a relatively low cell density as compared with the target cells, such that substantially all cells can interact with a target cells (there are more target cells available for the total number of cells comprised in the heterogenous cell population). Such provides for advantageous controllable conditions when applying the force on the cells, *e.g*. on the heterogeneous cell population.

With regard to the target cells and a cell clone with a receptor, it is understood that this may involve cells that are to have a specific interaction, *i.e.* one cell carrying a receptor and the other cell having a ligand for the receptor. For example, one cell may have a T cell receptor or a CAR-T, and the other cell may present an antigen for the T cell receptor, e.g. presenting an antigen via MHC. Such interactions are of interest to study and to modify. Hence, cells with a receptor, and control cells thereof not carrying *e.g*. the CAR, and target cells may be of particular of use in the means and methods of the invention, as exemplified in the example section herein. In a preferred embodiment immune effector cells are provided as cells with a receptor, and target cells expressing an antigen which the cell of interest is to specifically target. For example, effector cells may be selected from T cells, NK cells, dendritic cells, macrophages, or monocytes. Such cells may be genetically modified, *e.g*. provided with *e.g*. a CAR. As said, target cells are typically cancer cells, or other cells expressing an antigen, *e.g*. viral antigens or the like.

Further embodiments include methods being performed in the presence of agent(s). For example, one or more agents capable of modulating the cellular avidity between a cell with a receptor and a target cell may be included in the steps of contacting the heterogenous population of cells with the target cells and subsequently applying a force on the heterogenous population of cells away from the target cells. Modulating is understood to either enhance or reduce cellular avidity. This way, for example, in case an agent is present that is known to modulate a desired interaction between a cell with a receptor and a target cell, receptor/target interactions may be selected that are not affected by such agents, or, conversely, are aided by such agents. Hence, in another embodiment, an agent capable of modulating the interaction between the cell clones with a receptor and the target cell is included at least in the contacting step and when applying a force. For example, an agent could be used that reduces aspecific binding between a cell with a receptor and target cells. This may *e.g*. reduce the number of cells to be sequenced in case the remaining fraction is sequenced.

Of course, once receptor sequences with cellular avidity scores are provided, and these are ranked, from these rankings, receptor sequences may be selected for preparing a gene therapy vector. Of course, in scenarios wherein libraries of receptor sequences are tested, *e.g*. a CAR-T library, or wherein primary cells are provided comprising a variety of receptor sequences as well, the sequences identified may also be classified with regard to their properties, *e.g*. sequence homology or structural similarities. Hence, a ranking based on cellular avidity in addition to providing a sequence of a cell clone receptor may also include further information with regard to structure and/or sequence similarities. For example, receptor sequences may also be clustered due to sequence similarities. This way clusters may be identified that have similar and/or divergent cellular avidities, which may be highly useful in the selection process. Hence, in addition to ranking *e.g*. cell clones, cell clone clusters may be ranked as well, and moreover, a cellular avidity score may also be determined for a cell clone cluster. In this case, it may be possible for example to identify clusters of specific interest (*e.g*. clusters showing a higher than average avidity) based on counting of similar or related receptor sequences instead of identical receptor sequences. So, this method does not rely on multiple cells representing a cell clone in a cell population to be genetically identical but instead relies on multiple cells being similar.

In any case, based on the sequence information and cellular avidity information provided, and their ranking, subsequently, one or more receptor sequences may be selected and used to prepare *e.g*. a vector suitable for modifying an effector cell. Such vectors and modified effector cells may be ultimately used in therapeutic treatments. Hence, in a further embodiment in the method a receptor sequence is selected from the identified cell clone receptor sequences for preparing a gene therapy vector. Subsequently, a gene therapy vector is prepared which is subsequently in a final step admixed with a pharmaceutically acceptable buffer or otherwise pharmaceutical acceptably formulated.

It is understood that a synapse is a specialized structure that forms when the plasma membranes of two cells come into close proximity to transmit signals. Synapses can form between cells carrying a receptor and target cells, when *e.g*. the cells carrying the receptor are immune effector cells. Cells of the immune system form synapses that are essential for cell activation and function. Lymphocytes such as T cells, B cells and natural killer (NK) cells form synapses that can be referred to as immunological synapses. Such a synapse typically forms between effector cells and target cells, *e.g.* cells presenting an antigen. A non-limiting example is *e.g.* a T cell or a CAR-T cell and a cancer cell. The formation of synapses between *e.g*. an effector cell and a target cell, for example an APC (antigen presenting cell), is a hall-mark event and signals the presence of specific interactions (*i.e.* the specific interaction between, for example, a TCR or CAR and an antigen recognized thereby) between the effector cell and the target cell that are involved in the formation of such immunological synapses.

In the case of a T cell, a synapse can be formed between the lymphocyte and antigen-presenting cells (APCs) during the recognition of the peptide antigen-major histocompatibility complex (pMHC) ligand by the T cell antigen receptor (TCR). The TCR and pMHC are both membrane-bound, so the TCR will only be triggered by its ligand at the interface between T cells and APCs. A synapse can be observed at the T cell - APC interface as concentric rings by confocal microscopy, often referred to as "bull's eye" (Huppa, J. B., & Davis, M. M. (2003). T cell-antigen recognition and the immunological synapse. Nature Reviews Immunology, 3(12), 973-983). These rings were named the central, peripheral, and distal supramolecular activation cluster (*i.e.* respectively cSMAC, pSMAC and dSMAC). The TCR has been reported to be present in the cSMAC, whereas other lymphocyte specific proteins such as lymphocyte function-associated antigen-1 (LFA-1), are integrated into the pSMAC ring that surrounds the TCR. The formation of this ringed structure ("bull's eye") is however not universal and other formations such as "multifocal immunological synapses" between T cells and dendritic cells, or the like, have been described.

The immunological synapse can be considered to be any structure formed at the interface resulting from a functional and specific effector-target cell interaction, such as for example T cell - APC contacts. Markers associated with effector cells and synapse formation include one or more of CD43, CD44, CD45, LFA-1, Talin, F-actin, ZAP70, CD2, CD4, CD8, CD3, CD28, PD-1, ICOS, and TCR. Markers of target cells include one or more of ICAM-1 (associates with LFA-1), CD48/58 (interacting with CD2) CD80/CD86 (interacting with CTLA-4 and CD28), PDL1/PDL2 (associating with PD-1), and MHC presenting the antigen (that specifically interacts with the TCR). These markers, and concentrations thereof, may be detected *e.g*. with fluorescent labels at the interface between effector cell and target cell, or intracellularly in close proximity to the synaptic interface.

For example, markers of effector cells that have been associated with dSMAC are CD43, CD44, CD45. The effector cell markers LFA-1, Talin, F-actin, CD2, CD4 and CD8 have been associated with pSMAC. The markers CD3, CD28, PD-1, ICOS, and TCR of effector cells have been associated with cSMAC. Markers that have been associated with a synapse on a target cell are ICAM-1 (associates with LFA-1), CD48/58 (interacting with CD2) CD80/CD86 (interacting with CTLA-4 and CD28), and PDL1/PDL2 (associating with PD-1), and of course an MHC presenting the antigen (that specifically interacts with the TCR). These markers have been shown to be associated with synapses in a TCR-MHC interaction. Likewise, cell engagers (such as a bispecific antibody that *e.g*. binds CD3 on an effector cell with one arm and with the other arm an antigen on a target cell) which engage an effector cell with a target cell, can trigger the formation of a similar synapse structure as observed with a classic TCR-MHC interaction.

With regard to CARs of *e.g*. CAR T cells, these are chimeric antigen receptors that are to mimic a TCR or the like. CARs are engineered. The first generation of CAR were provided with an antigen recognition part often an antibody derived region (*e.g*. a scFv) fused to a transmembrane region and intracellular region of *e.g*. a CD3 ζ-chain. Later generations combined intracellular signalling domains from various costimulatory protein receptors (*e.g*., CD28, 41BB, ICOS) incorporated in the cytoplasmic tail of the CAR to enhance signalling further. Further generations also incorporated in their design an inducible release of transgenic immune modifiers, such as IL-12, to shape the tumor environment by augmenting *e.g*. T cell activation, attracting and activating innate immunity. As CARs often have antibody variable regions incorporated, these can target *e.g*. receptors themselves that are presented at the surface of a cell (*e.g*. Her2, PD-1 etc.), or can also target antigens presented by MHC, derived *e.g.* from proteins intracellular processed by the ubiquitin-proteasome system. Such peptides presented by MHC include proteins that are processed internally and presented by MHC, which can be derived from receptors, secreted proteins, intracellular proteins or internalized proteins. Synapses formed between CARs and target cells may not provide a classical bull's-eye like structure with a well-characterized SMAC domain, but may result in less organized pattern. Multiple CAR microclusters form and signalling molecules, which are dispersed in the center of the synapse interface.

Synapse formation is a spatiotemporal process that starts *e.g*. by TCR binding to MHC or binding of an antigen with CAR or cell engagement, and subsequent phosphorylation of the cytosolic tails of CD3 resulting in a triggered state. This sets of a cascade of processes that result in an activated T cell state, which also depends on the effector cell type and its phenotypic state. Key processes include: calcium signalling, internal cell structure and/or cytoskeleton changes of effector cells, involving F-Actin, Talin, and changes in microtubules, centrosomes, lytic granules, nucleus position, and mitochondrial location. Transactivation of adhesion molecules, cytokine and marker expression. IFNγ, granzyme and perforin may be released by effector cells to thereby induce *i.a*. target cell killing. Also, in addition, in target cells apoptotic markers can be found, including ICAM-1 clustering, phosphatidyl translocation, mitochondrial depolarization, caspase-3 activation and DNA fragmentation. Hence, various stages of specific effector cell and target cell interaction and immune activation can be detected.

In any case, synapse formation, or a synapse can be determined by staining, *i.e.* with fluorescent labels, ligands, antibodies, or probes, or the like, which may be used on live cells or on fixed cells targeting the mechanisms involved in T cell activation and/or synapse formation. Sequencing based methods may also be used to identify activated T cells and thereby associate mRNA levels with the formation of stable synapses. T cell activation leads to changes in mRNA stability and expression. *E.g*. increases in expression of cytokine or secretory transcripts (IL2, IFNγ, granzyme, perforin) and proliferation pathways and either bulk or single-cell RNA sequencing may be used to detect these changes and correlate these to the number or synapse formed.

In any case, in the cells obtained, which can be either in the form of target cells bound to effector cells or separated cells, *e.g*. with trypsin, the marker associated with synapse formation can be determined. Thus, in a further embodiment, the marker associated with synapse formation is determined in either the target cell or the effector cell. In another embodiment, in the methods in accordance with the invention one or more markers associated with synapse formation are determined and the one or more markers are determined in the effector cells and/or in the target cells. When cells are labelled, either in a singlet state or doublet state, cells may be highly advantageously be sorted and collected and subsequently analysed.

In one embodiment, a method in accordance with the invention is provided, wherein the marker associated with synapse formation is selected from the group consisting of calcium signalling signatures; spatial clustering of synapse localized molecules such as LFA-1, CD28, CD3, Agrin; changes to internal cell structure and/or cytoskeleton such as F-Actin, Talin, microtubules, centrosome, lytic granules, nucleus position, mitochondrial relocation; changes in effector cell motility; changes in external cell morphology and/or cell shape; and apoptosis of target cells. Synapse formation includes the initiation of a synapse up to and including the establishment of a synapse in which, as described above but not necessarily limited thereto, markers associated with synapse formation are associated.

In a further embodiment, the marker for synapse formation is calcium signalling, which can be detected with fluorescent calcium indicators, such as Fura2 AM (available from Invitrogen, item nr. F1221), which marker is suitable for detection in effector cells and in live cells. Other suitable dyes to detect calcium signalling can be selected from the group of Fura Red AM, Indo-1, pentapotassium, Fluo-3, fluo-4, Calcium Green-1, Rhod-2 and X-Rhod-1, Oregon Green 488 BAPTA. Hence, in one embodiment, the marker for synapse formation is calcium signalling which is detected with an indicator selected from the group consisting of Fura2 AM, Fura Red AM, Indo-1, pentapotassium, Fluo-3, fluo-4, Calcium Green-1, Rhod-2 and X-Rhod-1, and Oregon Green 488 BAPTA. With these markers calcium signalling can be detected which is a hallmark of synapse formation.

Furthermore, membrane potential dyes may also be used a calcium signalling indicators, such as the Invitrogen FluoVolt^{™} Membrane Potential Kit (Catalog number: F10488). Depolarization of the synapse forming cells by using a slow-response potential-sensitive probe such as Invitrogen DiSBAC2(3) (Bis-(1,3-Diethylthiobarbituric Acid)Trimethine Oxonol), Catalog number: B413. Hence, in another embodiment the marker for synapse formation is detected utilizing membrane potential dyes.

In another embodiment, the marker for synapse formation is cytoskeleton rearrangement, which can be detected in effector cells with live or fixed cells, using cell staining, *e.g*. F-actin can be detected with Phalloidin conjugates or CellMask^{™} (Invitrogen, item nr A57243). Other usable stains can include SiR-Actin, CellLight^{™} Talin-GFP, BacMam 2.0, or Tubulin Tracker Deep Red. Hence, in one embodiment, the marker for synapse formation is cytoskeleton rearrangement, which is detected with a stain selected from the group consisting of Phalloidin conjugates, CellMask ^{™}, SiR-Actin, Cell Light^{™} Talin-GFP, BacMam 2.0, or Tubulin Tracker Deep Red. With these markers, cytoskeleton rearrangement can be detected.

In another embodiment, the marker for synapse formation involves monitoring effector cell motility. Detection of effector cell motility can be performed by video/timelapse monitoring of effector cells that remain in contact with the target cells after the force has been exerted. Detection of synapse formation includes detecting effector cell immobility, *i.e.* upon synapse formation effector cells will stop moving and remain into contact with the target cell with which it forms a synapse. Cell motility can be detected by membrane staining of effector cells and imaging, or using brightfield, darkfield or phase contrast microscopy.

It is understood that for some of the methods for detecting a marker for synapse formation which use *e.g*. microscopy and monitoring of motility, or short-lived signals, may be combined with having cells provided with *e.g*. photoactivatable label, to, upon detection of the marker, activate such a label in the cell, such that in subsequent steps, *e.g*. sorting, FACS analysis or the like, cell for which the marker associated with synapse formation was detected can easily be tracked.

As described above, in accordance with the invention, markers associated with synapse formation can be determined, *e.g*. via utilizing labels or the like. However, it may be highly advantageous to sequence obtained cells and identify synapse formation by sequencing. It is understood that sequencing comprises nucleotide sequencing, *e.g.* sequencing of DNA and/or RNA as expressed in cells. Means and methods are widely known in the art to sequence DNA and/or RNA as expressed in the cell. Hence in another embodiment, in the methods in accordance with the invention the markers are determined with sequencing. This is in particular highly useful for such markers which are up- or downregulated in target cells and/or effector cells in forming a synapse or having a synapse. Suitable markers for T cell activation and synapse formation are transcripts linked to interferon expression, proliferation, and cytokine expression and include: Interferon pathway upregulation: CD4, IFIT3, IFIT2, STAT1, MX1, IRF7, ISG15, IFITM3, OAS2, JAK2, SOCS1, TRIM21; proliferation: LIF, IL2, CENPV, NME1, FABP5, ORC6, G0S2, GCK; cytokine expression: CCL3, IFNG, CCL4, XCL1, XCL2, CSF2, IL10, HOPX, TIM3, LAG3, PRF1, TNFRSF9, NKG7, IL26. Sequencing may also be used to identify non-synapse forming cells by detecting molecular signatures linked to resting cell states: FOXP3, CTLA4, MTNFRSF4, IRF4, BATF, TNFRSF18, TOX2, PRDMI, LEF1, ATM, SELL, KLF2, ITGA6, IL7R, CD52, S100A4, TGFB3, AQP3, NLRP3, KLF2, ITGB7.

It may be also advantageous to determine the molecular state of the target cells, as this can give an indication of the cell killing potency of the effector cells. In the case of paired analysis were effector-target T cell doublets are recovered, this enables direct linking of effector cell phenotype with their killing capabilities. Next to staining methods for apoptosis commonly used in the field, sequencing or qPCR can be used to detect transcripts linked to apoptosis or cell survival in the target cell, markers include: Bcl-2 family (BCL-xL) caspases 3, caspases 7, cleaved PARP, bax, bad, bak, bid, puma noxa, bcl-2, bcl-xl, mcl-1, p53, and cytochrome c, Smac/ Diablo, survivn, Mcl-1, RNA Y1.

As it is understood that changes in gene expression may take some time and are not necessarily immediately detectable, one may allow after the step of exerting the force, the cells to remain bound to the target cells for some time and have these remain attached to the surface as well. Alternatively, one may also separate *e.g*. doublets that remained after exerting the force and subsequently *e.g*. sort doublets in single wells, and allow these to remain for some time. A suitable time to allow for expression of markers associated with synapse formation may be from 1 hour to 24 hour.

This is highly advantageous as sequencing methods allow for single cell sequencing, which also allows for determining *e.g*. the sequences of receptors expressed by the cell as well. Hence, in a further embodiment, different receptors are determined and identified via sequencing. In yet another embodiment, sequencing comprises single cell sequencing. In still another embodiment, sequencing comprises sequencing the expressed genome. Sequencing the expressed genome is highly useful as it allows to determine up- and downregulated gene expression. Nevertheless, sequencing genomic DNA may be useful, as it may also provide useful information *e.g.* epigenetic markers associated with *in vivo* potency and durable responses.

With regard to the methods for determining cellular avidity of a plurality of cell clones with a receptor, capable of binding target cells, comprised in a heterogenous population of cells, it is understood that when the cells with a receptor, *i.e.* carrying a receptor, are immune effector cells, the methods may highly advantageously include determining the presence of a marker associated with synapse formation. This way, the cellular avidity scores that are to be determined in accordance with the invention can be based on the number of identified cells representing an identified cell clone and having a marker associated with synapse formation after applying the force. This way, the different fractions obtained may be defined further, *e.g*. defined effector cell clones that remain bound and defined effector cell clones that moved away from the target cells, may be further defined as effector cell clones that remain bound and have a synapse, effector cell clones that remain bound and not having a synapse and effector cells that moved away from the target cells. Accordingly, cellular avidity scores can be determined by calculating *e.g*. the ratio of the number of cells of an identified effector cell clone that were bound after applying a force and associated with a synapse to the number of cells of identified cell clones initially provided, or to the number of cells of identified effector cell clones that remained bound and did not form a synapse plus the identified effector cell clones that moved away from the target cells. In any case, as cell clones are highly preferably identified via sequencing, synapses may be identified via sequencing methods. Of course, one may also simply sort cells associated with a synapse, from cells not associated with a synapse.

It was observed that when cells that remained bound and attached after a cellular avidity measurement were resuspended utilizing *i.a*. repeated pipetting and thus exerting a significant force, cells attached to the surface were detached and moreover, a portion of the cells that were bound to the attached cells became unbound. Hence, this implied that with such a process step a differential force can be applied on bound cells that can exceed the maximum force that is applied away from cells attached to a surface. This way, further cell-cell bonds that may be aspecific cell-cell bonds may thus be broken therewith providing substantially specific cell-cell bonds that remain, *e.g*. effector cells bound with target cells (and optionally a cell engager) that formed a synapse can be retained. Hence, in a further embodiment, after the force has been applied away from the attached cells, subsequently, the cells are resuspended and a differential force is applied such that substantially effector cells that are bound to each other via a synapse remain bound to each other and substantially cells that have formed aspecific bonds are unbound, *i.e.* have their cell-cell bonds broken. It is also understood that instead of applying the force away, the differential force may be applied instead, and that by applying a differential force, it may no longer be required to have cells attached to a surface, *i.e.* immobilized. Furthermore, it is also understood that after the differential force has been applied, cells are highly preferably separated and/or sorted in order to avoid further interaction between cells to avoid establishing additional cell-cell bonds. Hence, highly preferably, cells are collected and sorted and/or analysed after the differential force has been applied. This way, fractions of cells can be obtained with which, in analogy and likewise to the situation as described above when applying a force away from attached cells, cellular avidity scores can be determined of identified cells, *e.g*. optionally combined with determining the presence of markers associated with synapses.

As is clear from the above, the type of force that is to be applied in accordance with the invention is a force capable of breaking cell-cell bonds, *i.e.* the force exerted causes cells bound to each other move away from each other to such an extent that a cell-cell bond may break or rupture. A differential force means that the force on one cell differs from the force on the other cell with regard to direction of the force and/or the magnitude of the force, resulting in a net force allowing to break cell-cell bonds if the differential force exceeds the binding force.

For example, when a target cell bound to an effector cell, a doublet, is forced through a nozzle, the closer to the throat of the nozzle the faster the flow. This means that the first cell to enter the nozzle is subjected to a stronger acceleration than the cell lagging behind and the cells experience a differential force resulting in a net force which can result in cell-cell bond rupture, provided the force is large enough (see *e.g*. Figure 5, in particular 5d). A differential force that can be applied includes a shear force, *e.g*. such as can be applied utilizing repeated pipetting (repeated upwards and downwards flow of the sample) or flow through a nozzle.

Other means and methods are known in the art with which shear forces can be applied to cells, *e.g*. flowing a cell suspension at a constant speed and bombarding these cells to a flat surface at a defined angle. Furthermore, forcing a cell suspension through a needle with a defined internal diameter and a defined force may provide for a well controllable shear force as well. The cell suspension may be subjected to several rounds of such process steps to ensure substantially all cell-cell bonds experience the maximum force that may be achieved with the process step. Such a process step allows for automation, enabling control and repeatability of the process, therewith controlling shear forces exerted. Suitable devices for breaking apart cell-cell bonds which are not synapses are known in the art (*e.g.* Zahniser et al., J Histochem Cytochem. 1979, 27 (1), 635-641). Also, by properly tuning the forces in a flow-cytometer normally used to measure cell deformations, such as *e.g.* described in Otto, et al. Nat. Methods 12, 199-202 (2015) suitable forces can be applied. Tuning can be achieved *e.g*. by changing the nozzle size or geometry and/or the flow speeds used. Other suitable devices known in the art may include for a vortex mixer, with which shear forces may be suitably applied as well. Accordingly, in one embodiment, the force applied involves a shear force.

In another embodiment, the force applied is an ultrasonic force. It is understood, as outline above, that such ultrasonic forces are not forces such as applied *e.g*. in a device as available from Lumicks, wherein the force is away from attached cells (*e.g*. such as in the LUMICKS z-Movi^{®} Cell Avidity Analyzer, *e.g.* as used by Larson et al., Nature 604 7906:1-8, April 13, 2022). It is also understood that the ultrasonic force is selected such that cells are not lysed. Hence, appropriate ultrasonic forces can be applied to cells such that cell-cell bonds can be ruptured, which more preferably includes breaking aspecific cell-cell bonds and less preferably breaks specific cell-cell bonds in which an immune synapse is formed. Examples of using ultrasonic forces to break (aspecific) cell-cell bonds, are known in the art (*e.g.* as described in Buddy et al., Biomaterials Science: An Introduction to Materials in Medicine, 3rd edition, 2013, Chapter II.2.8, page 576; and Moore et al., Experimental Cell Research, Volume 65, Issue 1, 1971, Pages 228-232).

In any case, suitable applied forces which are known in the art include *e.g*. a force in the range of 50 pN - 10 nN, which said force is a net force exerted on one cell relative to the other cell, of two cells bound to each other. Which means the force is exerted on the cell-cell bond. In another embodiment, the force exerted on one of the two cells relative to the other cell is at least 50 pN, or at least 100 pN, or at least least 200 pN. In another embodiment, the force exerted is at most 10 nN, at most 5 nN, at most 3 nM, at most 2 nM, or at most 1 nN. In yet another embodiment, the force is selected from the range of 1 pN - 10 nN, from 100 pN - 10 nN, from 500 pN - 10 nN, from 1 nN - 10 nN. In still a further embodiment, the force is selected from the range of 500 pN - 5 nN, from 500 pN - 4 pN, from 500 pN - 3 pN. For example, a suitable amount of force that can be exerted between cells (*e.g*. such as in the z-Movi^{®} device) can be selected to be in the range of 200 pN - 3000 pN. Of course, these force ranges are known to be useful with cells attached to a surface, and the maximum force that may be selected may exceed 3000 pN as it may not be required to have the cells attached to a surface.

Accordingly, it is understood that in these embodiments, the differential force to be applied does not require either of the target cells or the plurality of cell clones with a receptor, capable of binding target cells, comprised in a heterogenous population of cells, *e.g*. effector cells, to be attached, and the differential force can be a force selected from the range of 50 pN - 10 nN. In another embodiment, in methods in accordance with the invention wherein the force that is applied is a differential force, neither the target cells nor the cells with a receptor, *e.g*. effector cells, require to be attached to a surface, and the differential force is applied in the range of 50 pN - 10 nN, thereby providing cells substantially comprised of target cells, cells with a receptor, and cells with a receptor bound to target cells. It is understood that by selecting an appropriate force, aspecific cell-cell bonds may be selectively broken, while retaining specific cell-cell bonds between cells with a receptor and target cells. For example, in case cells with a receptor are effector cells, effector cells bound with target cells via a synapse may be retained.

Without being bound by theory, as is understood the range of force that may break an aspecific cell-cell bond versus a specific cell-cell bond that forms a synapse differs. This difference can be to such an extent that the ranges of the required forces do not overlap. It is understood that some overlap may occur. Hence the force that is selected, as outline above, may allow for aspecific cell-cell bonds remaining and some specific cell-cell bonds that formed a synapse to break. In case there is substantially no overlap, a differential force can be selected, as outline above, which allows substantially for aspecific cell-cell bonds to break, while substantially retaining specific cell-cell bonds that formed a synapse. In case there is no overlap, and ranges are sufficiently far apart, a differential force may be selected, as outline above, which allows for aspecific cell-cell bonds to break while retaining specific cell-cell bonds that formed a synapse. As also outlined herein, the portion of cell-cell bonds that remains after exerting a force and has a synapse, can be determined by determining the presence or absence of a marker associated with synapse formation. For example, by performing the method with a reference immune effector cell with a receptor, and a target cell, a suitable force may be selected with which to carry out the methods of the invention, *e.g*. wherein a suitable force results in high percentage of synapses retaining. Likewise the same principles can be applied for further cells carrying a receptor and target cells.

In one embodiment, after the step of applying a force away from attached cells in the methods of the invention, the cells are resuspended and a subsequent differential force is applied such that formed aspecific cell-cell bonds are broken. In another embodiment, in methods of the invention wherein either the target cells or the heterogenous population of cells are attached to a surface, this attachment is optional, and, in the step of applying the force instead a differential force is applied. In yet another embodiment, in methods of the invention wherein the heterogenous population of cells comprises immune effector cells, either the target cells or the heterogenous population of cells are attached to a surface, this attachment is optional, and, in the step of applying the force instead a differential force is applied. In a further embodiment, said heterogenous population of cells comprises immune effector cells and said differential force is such that cells that are bound via a synapse remain bound to each other and formed aspecific cell-cell bonds are broken.

### Examples

### Example 1

With regard to separation of different cell clone populations, the concept of separating the cells utilizing cellular avidity separation methods can be assessed. For example, a heterogeneous cell population comprising 5 different cell clones having different cellular avidities is provided. Comprising cell clones A, B, C, D and E, each having a cellular avidity of 95%, 75%, 50%, 25% and 10%, respectively. When such a heterogeneous cell population, consisting of 1000 cells, wherein each cell clone is present in specified amounts (w, x in table below), would be subjected to a cellular avidity separation and analysis, what would be the result? Below in Table 1 the result is shown that would be obtained when fractions of detached cells (y) and cells that remained attached (z) would be collected and identified. In Table 1 below, the % of cells at the start is listed, which can be determined *e.g*. by (sequence) analyzing a representative sample therefrom at the start, or calculating it from the total number of cells that were contacted with the target cells *e.g*. by adding detached and attached cell numbers.

From Table 1, it is clear the number of cells attached or detached, or percentages thereof (see columns y, z, q and r) are not, by themselves, indicative for cellular avidity, which in this case, is known *a priori.* Hence, some calculations are needed from the results obtained that relate to cellular avidity. We can calculate for example the ratio of cells attached by cells at the start (z/x) to provide for a cellular avidity score. This number may also be expressed as percentages. This number can also be calculate based on the detached number of cells (1-y/x). This may also be referred to as an inferred absolute cellular avidity, as opposed to relative cellular avidity (see below).

We can also calculate the relative cellular avidity, if we for example do not know the number of cells that interacted with the cell target layer. This number can be calculated by calculating the ratio of % of cells detached divided by the % of cells at the start (r/w). This number also reflects the fold enrichment obtained in the fraction. One can also calculate the ratio of cells attached by the cells detached (r/q or z/y). Besides cellular avidity, this number also indicates the fold enrichment of the attached fraction relative to the detached fraction. The results of these calculations are listed below in Table 2. Whereas in Table 1 no ranking could be made, the calculated (relative/absolute) cellular avidity scores as shown in Table 2 rank as they should.

It is shown that when the composition of at least two fractions is either known or determined, (relative) cellular avidity can be determined which allows ranking identified cell clones, and their corresponding receptors, accordingly. Hence, it is shown that when cell clones are identified, *e.g*. by sequencing receptor sequences, and quantified, from heterogenous pools, one can easily determine cellular avidity and rank cell clone receptors accordingly.

### Example 2

The previous example illustrates how identifying cell clones after cellular avidity sorting/selection can be used to obtain cellular avidity information from a heterogeneous cell sample without prior knowledge of the cell sample and without having access to individual cell clones prior to the sorting/selection step to allow identifying different cell clones of interest. Although that example illustrates how identification of cell clones post sorting (*e.g*. by quantitative sequencing of unbound and/or bound cells) a plurality of clones can be identified and ranked according to their varying avidity (*e.g*. 5 cell clones can be ranked from a sample containing 5 or more clones). In practice there may often be a large number of aspecific cells (*e.g*. untransduced cells and/or cells that are not specific to the antigen presented on the target cells and/or functionalized wall surface.

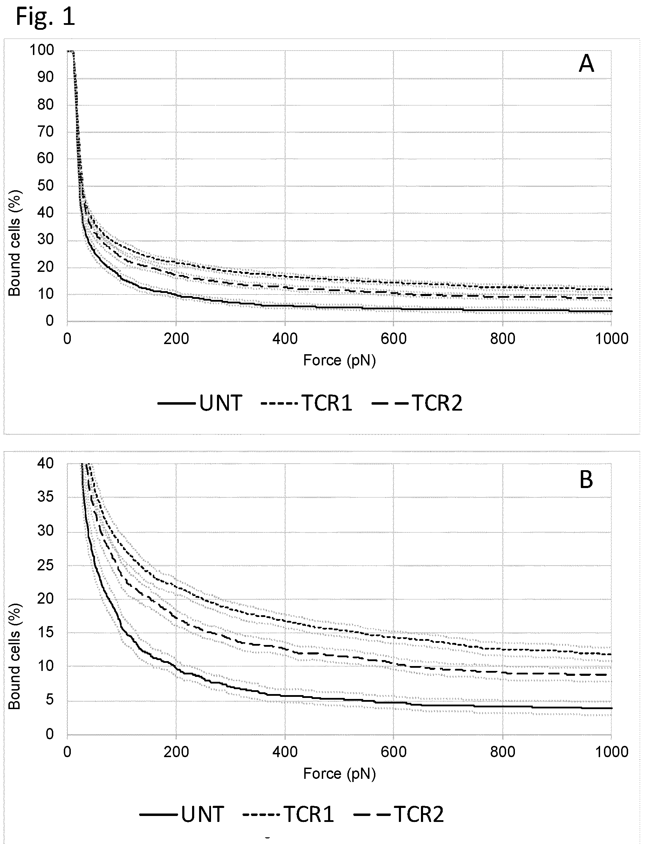

To illustrate how the presence of *e.g*. a majority of untransduced cells may impact the analysis we performed another *in-silico* analysis utilizing actual data generated from the z-Movi^{®} analyzer. Figures 1A and 1B summarize cellular avidity experiments obtained using the z-Movi^{®} acoustic avidity analyzer.

### Primary cells and cell lines

Primary T cells were obtained from a healthy donor and Magnetic-activated cell sorting assay (MACS) was used to enrich for CD8 positive cells. Enrichment was verified using fluorescence assisted cell sorting (FACS) and cells were either used as untransduced cells or first transduced using a retrovirus with one of two TCR sequences (TCR1 and TCR2) specific for an antigen presented by the target melanoma cell line. The transduction rates ranged from 21% to 57%. Cultures were then expanded and enriched for expression of the introduced TCRs using MACS. The final T cell populations were 89 - 96% TCR positive as determined by FACS. These cells were used as the effector cells. A melanoma cell line was used as target cell layer.

### Cellular Avidity measurement

Z-Movi^{®} chips (obtained and as available from LUMICKS (https://lumicks.com/products/z-Movi^{®}-cell-interaction-studies/)), were coated the day before performing the experiment with Poly-L-Lysine (#P4707-50ML, Sigma) diluted 1:5 in PBS for 10 minutes. The following morning the target cells were seeded in serum-free culture medium at a density of 50 million/mL, incubated for 1h at 37°C, and the medium was replaced with advanced RPMI complete medium. The target cells were incubated for 4 h before starting the cellular avidity experiment. Freshly thawed T cells (effector cells) rested for 2 hours in T cell medium (TCM) before avidity measurements. This medium consisted of 1640 RPMI, 10% FBS, 1 mM sodium pyruvate, 1x non-essential amino-acids, 50 µM 2-mercaptoethanol, and 100 IU/mL penicillin and streptomycin. The effector cells were stained with CellTrace^{™} Far Red dye (Thermo Fisher Scientific) at 1 µM for 15 minutes in PBS at 37°C, then resuspended at 10 million/mL in complete medium and used for the avidity experiments. The effector cells (*i.e.* cells of interest also referred to as cells with a receptor) were introduced in the target cell-seeded flow cell, incubated for 10 minutes, and then a force ramp from 0 to 1000 pN was applied in 2.5 minutes.

For all three effector cell types a total number of 10 avidity curves was measured over a period of 4 days using three different z-Movi^{®} chips.

Figures 1A and 1B show the percentage of cells bound to the target cell layer as a function of the applied force. The black solid or dashed lines show the mean from the 10 runs. The standard error of the mean (SEM) is indicated as the grey dots surrounding each mean. Figure 1B is a zoom-in of Figure 1A and thus more clearly shows the differences in the avidity curves that occur at higher forces. We can see that the upper line (highest avidity) is for TCR1 with a percentage bound at 1000 pN of 12±1 % (mean±SEM). TCR2 shows a binding at 1000pN of 9±1 % and the untransduced cells show a binding at 1000pN of 4±1 %. We can interpret these mean percentage of cells bound at 1000pN as the avidity scores for each cell type.

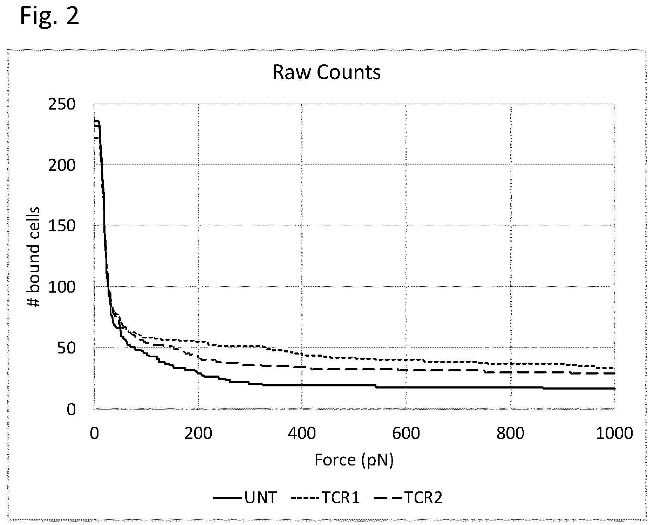

Figure 2 shows the raw cell counts (number of bound cells as a function of applied force) for each cell type for a single run (out of the 10 repeat runs). In this case the untransduced (UNT) cell experiment starts with 231 cells bound to the target cell layer and ends with 16 cells that remain bound at 1000pN. For TCR1 the number of cells at the beginning and end are 222 and 33 respectively. For TCR2 the number of cells at the beginning and end are 236 and 29 respectively. Based on these individual curves we would assign avidity scores as follows: UNT: 16/231 = 7%, TCR1: 33/222 = 15%, TCR2: 29/236 = 12%. This illustrates that although for this specific random selection of runs the cellular avidity scores are not the same as the averaged results from Figure 1. However, the ranking of the cellular avidity scores here is the same with UNT < TCR2 < TCR1 which indicates that reasonable cellular avidity estimates can be obtained.

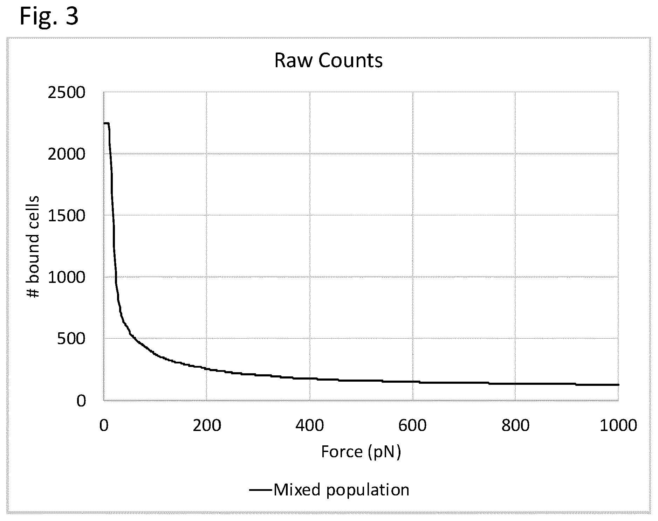

Figure 3 shows a hypothetical avidity experiment where we summed up all 10 runs of the untransduced cells from Figure 1 together with the single runs for TCR1 and TCR2 displayed in Figure 2. This leads to a simulated experiment with 1784 cells with random TCRs (the untransduced cells) and 222 cells with TCR1 and 236 with TCR2. This artificial sum thus simulates an experiment with a heterogeneous sample containing 80% cells with random unspecific TCR sequences and low numbers of clones per type, 10% clones of type TCR1 and 11% of clones of type TCR2 (numbers are rounded). At the start of the run (0 pN) we have all cells together (2242 cells) contacting the functionalized wall surface. After ramping the force to 1000 pN, and optionally sampling different fractions of unbound cells at various forces in between, we would be left with 123 cells bound to the surface. After sequencing these cells, we would find there are 69 cells with different random TCR sequences and 33 cells with sequence TCR1 and 29 cells with sequence TCR2. If we then check for the relative abundance of these two sequences with high abundance in the remaining fraction in a sample of 2000 cells from the starting population, we have a 95% chance of finding between 174 and 228 cells of type TCR1 and between 193 and 249 cells of type TCR2 (since the selection is a random sampling with a 10% chance of selecting TCR1 and an 11% chance of selecting TCR2). We assume a binominal distribution for the selection process. Based on this and the knowledge we started out with a total number of 2242 cells in the sorter, we would then normalize the amount of TCR1 and TCR2 sequences found in the end and estimate an avidity score of 0.13 to 0.17 for TCR1 and an avidity score of 0.10 to 0.13 for TCR2. This should be compared to the scores of 0.15 and 0.12 obtained by the direct measurement on pure TCR1 and TCR2 cells (see Figure 2). For larger cell numbers (both in the avidity sorted data and in the normalization sample) these estimates would obviously improve. *E.g*. in the current case checking the abundance of TCR1 and TCR2 in a sample of 20000 cells would lead with 95% confidence to estimates of 0.14 to 0.16 for TCR1 and an avidity score of 0.11 to 0.12 for TCR2 without changing the number of cells being sorted. If we increase the number of sorted cells as well even better estimates can be obtained.

This example illustrates good avidity estimates can be obtained even from unknown heterogeneous samples containing large numbers of aspecific cells provided there are a reasonable number of cell clones of the specific cells of interest (*e.g.* of the specific TCRs that bind with higher avidity to the tumor cells than the aspecific TCRs).

### Example 3

### Avidity determination and ranking of CAR-T effector cell populations by means of RNA sequencing

Two effector cell populations were prepared from primary human T cells by retroviral transduction with two different CAR constructs (construct A and construct B), resulting in CAR-A, and CAR-B with known high and medium avidity, respectively. A third population (UNT) of untransduced T cells was also prepared to serve as a control.

To show avidity fractionation the following experiment was conducted. In the experiment a channel slide (Ibidi) was used. The slide contained a flow channel extending between a channel inlet at an opening side and a channel outlet at the opening side and defining a ceiling associated with the opening side and a floor opposite the ceiling.

A monolayer of NALM6 (CD19+) target cells was attached to the ceiling of a 200 µm high fluidic channel obtaining a confluence close to 100% by means of the following procedure.

The Nalm6 (CD19+) cells (obtained from ATCC, product nr. CRL-1567) were grown, harvested and resuspended in serum free RPMI1664 medium. Hereafter, the fluidic channel of a slide (Ibidi), which channel was coated the day before performing the experiment with poly-L-Lysine (PLL, Sigma) diluted 1:5 in PBS for 10 minutes, was seeded with 80x10⁶ cells/ml and the slide was flipped and incubated upside down for 30 minutes in humid incubator at 37°C. After incubation, the slide was flipped and the medium in the channel was exchanged twice with 150 µl RPMI1664 medium supplemented with 10% fetal calf serum (FCS). The slide was incubated upside down again for 30 minutes in humid incubator at 37°C. Thereafter, to test monolayer integrity, the slide was flipped to put it in an upright position and it was centrifuged at 10xg for 2 minutes, washed and a visual check of monolayer integrity was performed using a table top microscope. The slide was stored upside down in a humid incubator at 37°C until use.

A mixed effector cell sample Input=(33.3% CAR-A, 33.3% CAR-B, 33.3 UNT) was resuspended in complete RPMI medium and introduced in the channel at a concentration of 7e6/ml. The channel was turned upside down to allow the effector cells to contact the monolayer on the channel ceiling. The effector cells were then incubated on top of the monolayer for 5 minutes in a humid incubator at 37°C. Next, the slide was flipped back to its upright position and centrifuged at 1000xG for 2 minutes. Unbound cells were removed from the channel by flushing the channel 4 times with 100 µl of serum containing medium. Cells were counted and a fraction of 50,000 cells (sample called Unbound) was placed into a clean 1.5 ml tube and pelleted and resuspended in TRIzol (ThermoFisher 1234), and placed at -20°C until further use. In total 7 samples were prepared and stored in TRIzol: 50,000 cell pure fractions of: CAR-A, CAR-B, Untransduced (UNT), NALM6 (Mono), the mixed input sample Input=(33.3% CAR-A, 33.3% CAR-B, 33.3 UNT). And two recovered fractions: Unbound (recovered after centrifugation of the Input sample), Bound (recovered cells from inside the channel composed of monolayer target cells and effector cells that remained attached to the monolayer after centrifugation of the Input sample).

Bulk RNA Sequencing libraries were prepared from all cell fractions by Single Cell Discoveries, according to an adapted version of the SORT-seq protocol (Muraro et al., Cell Syst. 26;3(4):385-394, 2016) with primers described in van den Brink et al., Nature Methods 14:935-936, 2017). RNA was extracted from TRIzol and used for cDNA synthesis using barcoded primers. After second-strand cDNA synthesis, the samples were pooled and amplified using *in vitro* transcription (IVT). Following amplification, library preparation was done following the CEL-Seq2 protocol (Hashimshony et al., Genome Biology 17: article number:77, 2016) to prepare a cDNA library for sequencing using TruSeq small RNA primers (Illumina). The DNA library was paired-end sequenced on an Illumina Nextseq^{™} 500, high output, at 20 million raw reads per sample (1x75).

RNA Sequencing analysis was performed by Barbanson Biotech. The raw reads of ten bulk RNA-seq libraries were trimmed and mapped with STAR (https://github.com/alexdobin/STAR, default settings) to a constructed HG38 reference file including the CAR retroviral vectors. Next, molecules are assigned to genes, quality filtered and de-duplicated using SingleCellMultiomics1 bamtagmultiome.py (https://github.com/BuysDB/SingleCellMultiOmics).

To estimate the cell ratios in the recovered fractions all samples were first normalized using a set of transcripts expressed homogeneously across all samples. To estimate the mixing ratios a two-step procedure was used. First marker genes for the monolayer, untransduced, and the combined CAR expressing cells were identified by differential analysis of the pure input cell samples. Cell fractions were subsequently calculated from the proportion of marker genes detected in each sample. Second, the fraction of CAR-B expressing cells was estimated by calculating the fraction of uniquely mapping CAR-B reads versus uniquely mapping CAR-A reads. This is calculated as: fraction CAR-B = CAR-B / (CAR-B + CAR-A).The predicted fractions are listed Table 5.

The data show that transcriptomic sequencing can be used to infer avidity and correctly differentiate between CAR-T effector cells of different avidity. Only considering the absolute bound or unbound fractions is insufficient (see Table 5 (r and q)), but calculating the relative avidity as r/Input sample correctly ranks the CAR-A, CAR-B and UNT as high, medium and low avidity. As expected the Input sample had approximately equal population of CAR-A, CAR-B and UNT effector cells. The unbound fraction after centrifugation showed a small reduction in the high avidity CAR-A population. It also included a small fraction of monolayer cells that were also collected in the recovery of the cells. The recovered bound fraction was dominated by the collected monolayer cells but showed a clear enrichment of the high avidity CAR-A population markedly higher than the medium avidity CAR-B.

**Table 1: Cellular avidity data.**

| | Cellular avidity | % Cells Start | # Cells start | Cells detached | Cells attached | % in detached | % in attached |
|---|---|---|---|---|---|---|---|
| | | w | x | y | z | q | r |
| A | 95% | 10% | 100 | 5 | 95 | 1% | 21% |
| B | 75% | 30% | 300 | 75 | 225 | 16% | 42% |
| C | 50% | 10% | 100 | 50 | 50 | 11% | 9% |
| D | 35% | 15% | 150 | 97 | 53 | 21% | 10% |
| E | 10% | 35% | 350 | 315 | 35 | 67% | 7% |
| Total | | | 1000 | 542 | 458 | | |

**Table 2: Calculations of cellular avidity.**

| | Cellular avidity | Absolute Cellular avidity | Relative cellular avidity | Relative cellular avidity |
|---|---|---|---|---|
| | | z/x | z/y | r/w |
| A | 95% | 0.95 | 19.00 | 2.07 |
| B | 75% | 0.75 | 3.00 | 1.64 |
| C | 50% | 0.50 | 1.00 | 1.09 |
| D | 35% | 0.35 | 0.55 | 0.77 |
| E | 10% | 0.10 | 0.11 | 0.22 |

**Table 3.**

| | Cellular avidity * | % cells at start ** | # cells at start | Cells detached | Cells attached | % in detached | % in attached |
|---|---|---|---|---|---|---|---|
| | | w | x | y | z | q | r |
| UNT | 4 | 78 to 81 % | not known | 1723 | 61 | 78 | 50 |
| TCR1 | 12 | 9 to 11 % | not known | 189 | 33 | 9 | 27 |
| TCR2 | 9 | 10 to 12 % | not known | 207 | 29 | 9 | 24 |
| Total | | | 2242 | 2219 | 123 | | |

| | | | | | | | |
|---|---|---|---|---|---|---|---|
| * cellular avidity of the cell types as determined based on the averaged data shown in Figure 1. ** 95% confidence interval of the expected relative abundance determinations based on a random sampling of 2000 cells from a population with the relative abundances as discussed in relation to Figure 3. | | | | | | | |

**Table 4: Cellular avidity calculations.**

| | Cellular avidity | Absolute Cellular avidity | Relative cellular avidity | Relative cellular avidity |
|---|---|---|---|---|
| | | z/(z+y) | z/y | r/w |
| UNT | 4% | 0.034 | 0.035 | 0.62 - 0.64 |
| TCR1 | 12 % | 0.15 | 0.17 | 2.5 - 3.0 |
| TCR2 | 9% | 0.12 | 0.14 | 2.0 - 2.4 |

**Table 5: Sequencing based prediction of avidity separated cellular fractions.**

| | CAR-A | CAR-B | UNT | Mono |
|---|---|---|---|---|
| Input sample | 24.1% | 30.0% | 45.8% | 0.1% |
| Unbound sample | 21.3% | 35.7% | 36.6% | 6.4% |
| Bound sample | 8.6% | 1.7% | 2.0% | 87.6% |
| % Attached (r) | 69.9% | 13.9% | 16.2% | n.a. |
| % Detached (q) | 22.7% | 38.2% | 39.1% | n.a. |
| Relative cellular avidity (r/Input sample) | 2.90 | 0.46 | 0.35 | n.a. |

## Claims

1. A method for determining the cellular avidity of a plurality of cell clones (6) with a receptor (5), capable of binding target cells (2), comprised in a heterogenous population of cells by:
a) providing a heterogenous population of cells comprising a plurality of cell clone populations carrying a receptor;
b) providing target cells attached to a surface (1);
c) contacting the heterogenous population of cells with the target cells;
d) applying a force (7) on the heterogenous population of cells away from the target cells;
e) collecting detached cells to provide for at least one cell fraction;
f) identifying cell clones comprised in the fraction(s) provided in step e), and determining the number of cells representing identified cell clones in the fraction;
g) assigning cellular avidity scores to identified cell clones based on the determined numbers of step f).

2. The method in accordance with claim 1, wherein in addition to, or instead of, collecting the detached cells, cells that remain attached to the target cells at the end of the applied force are collected as a fraction in step e).

3. The method in accordance with claim 1 or 2, wherein of the heterogenous population of cells provided in step a) a fraction is collected of the provided starting population in step a) which represents a further fraction provided in step e); and/or wherein the heterogenous population of cells provided in step a) is defined with regard to cell clone populations comprised therein.

4. Method in accordance with any one of claims 1 to 3, wherein in addition a defined portion of control cells and/or reference cells is included in the heterogeneous population of cells provided in step a).

5. The method in accordance with any one of claims 1 to 4, wherein the number of cells representing a cell clone is determined by quantifying nucleic acid sequences representing a cell clone.

6. The method in accordance with any one of claims 1 to 5, wherein for an identified cell clone, the percentage of cells that remained bound relative to the initial cell clone population comprised in the heterogenous population is calculated as a cellular avidity score.

7. The method in accordance with any one of claims 1 to 6, further comprising ranking identified cell clones and receptor sequences based on avidity scores.

8. The method in accordance with any one of claims 1 to 7, wherein the applied force is a force ramp, preferably a linear force ramp.

9. The method in accordance with any one of claims 1 to 8, wherein the applied force is an acoustic force, a shear flow force or an acceleration force.

10. The method in accordance with any one of claims 1 to 9, wherein the target cells are attached to a glass or plastic surface, preferably as a monolayer.

11. The method in accordance with any one of claims 1 to 10, wherein the target cells are cancer cells or cells presenting a cancer antigen.

12. The method in accordance with any one of claims 1 to 11, wherein the cells with a receptor are immune effector cells.

13. The method in accordance with claim 12, wherein the effector cells are selected from T lymphocytes, NK cells, monocytes, macrophages and dendritic cells.

14. Method in accordance with any one of claims 1 to 13, wherein the receptor is a CAR, a TCR, a stimulatory or a inhibitory coreceptor.

15. Method in accordance with any one of claims 1 to 14, wherein an agent capable of modulating the interaction between the cell clones with a receptor and the target cell is included in step c) and subsequent steps.

## Patentansprüche

1. Verfahren zum Bestimmen der Zellavidität mehrerer Zellklone (6) mit einem Rezeptor (5), der in der Lage ist, Zielzellen (2) zu binden, die in einer heterogenen Population von Zellen enthalten sind, durch:
a) Bereitstellen einer heterogenen Population von Zellen, die mehrere einen Rezeptor tragende Zellklonpopulationen umfasst;
b) Bereitstellen von Zielzellen, die an eine Oberfläche (1) gebunden sind;
c) In-Kontakt-Bringen der heterogenen Population von Zellen mit den Zielzellen;
d) Anlegen einer Kraft (7) an die heterogene Population von Zellen weg von den Zielzellen;
e) Sammeln abgelöster Zellen, so dass für mindestens eine Zellfraktion gesorgt wird;
f) Identifizieren von Zellklonen, die in der/den in Schritt e) bereitgestellten Fraktion(en) enthalten sind, und Bestimmen der Zahl von Zellen, die identifizierte Zellklone in der Fraktion repräsentieren;
g) Zuweisen von Zellaviditätswertungen an identifizierte Zellklone aufgrund der bestimmten Zahlen aus Schritt f).

2. Verfahren gemäß Anspruch 1, wobei zusätzlich zu oder anstelle von Sammeln der abgelösten Zellen Zellen, die am Ende der angelegten Kraft an den Zielzellen gebunden bleiben, als Fraktion in Schritt e) gesammelt werden.

3. Verfahren gemäß Anspruch 1 oder 2, wobei von der in Schritt a) bereitgestellten heterogenen Population von Zellen eine Fraktion der in Schritt a) bereitgestellten Ausgangspopulation gesammelt wird, die eine weitere in Schritt e) bereitgestellte Fraktion darstellt; und/oder wobei die in Schritt a) bereitgestellte heterogene Population von Zellen in Bezug auf darin enthaltene Zellklonpopulationen definiert ist.

4. Verfahren gemäß einem der Ansprüche 1 bis 3, wobei zusätzlich ein definierter Teil von Kontrollzellen und/oder Referenzzellen in der in Schritt a) bereitgestellten heterogenen Population von Zellen enthalten ist.

5. Verfahren gemäß einem der Ansprüche 1 bis 4, wobei die Zahl der Zellen, die einen Zellklon repräsentieren, durch Quantifizieren von Nukleinsäuresequenzen, die einen Zellklon repräsentieren, bestimmt wird.

6. Verfahren gemäß einem der Ansprüche 1 bis 5, wobei für einen identifizierten Zellklon der Prozentsatz von Zellen, die gebunden blieben, bezüglich der anfänglichen Zellklonpopulation, die in der heterogenen Population enthalten ist, als Zellaviditätswertung berechnet wird.

7. Verfahren gemäß einem der Ansprüche 1 bis 6, ferner umfassend Erstellen einer Rangliste identifizierter Zellklone und Rezeptorsequenzen bezogen auf Aviditätswertungen.

8. Verfahren gemäß einem der Ansprüche 1 bis 7, wobei es sich bei der angelegten Kraft um eine Kraftrampe, vorzugsweise eine lineare Kraftrampe, handelt.

9. Verfahren gemäß einem der Ansprüche 1 bis 8, wobei es sich bei der angelegten Kraft um eine akustische Kraft, eine Scherströmungskraft oder eine Beschleunigungskraft handelt.

10. Verfahren gemäß einem der Ansprüche 1 bis 9, wobei die Zielzellen an einer Glas- oder Kunststoffoberfläche, vorzugsweise als Monoschicht, befestigt sind.

11. Verfahren gemäß einem der Ansprüche 1 bis 10, wobei es sich bei den Zielzellen um Krebszellen oder Zellen, die ein Krebsantigen präsentieren, handelt.

12. Verfahren gemäß einem der Ansprüche 1 bis 11, wobei es sich bei den Zellen mit einem Rezeptor um Immuneffektorzellen handelt.

13. Verfahren gemäß Anspruch 12, wobei die Effektorzellen aus T-Lymphozyten, NK-Zellen, Monozyten, Makrophagen und dendritischen Zellen ausgewählt sind.

14. Verfahren gemäß einem der Ansprüche 1 bis 13, wobei es sich bei dem Rezeptor um einen CAR, einen TCR, einen stimulierenden oder einen hemmenden Corezeptor handelt.

15. Verfahren gemäß einem der Ansprüche 1 bis 14, wobei ein Agens, das in der Lage ist, die Wechselwirkung zwischen den Zellklonen mit einem Rezeptor und der Zielzelle zu modulieren, in Schritt c) und nachfolgenden Schritten enthalten ist.

## Revendications

1. Procédé de détermination de l'avidité cellulaire d'une pluralité de clones cellulaires (6) avec un récepteur (5), capable de se lier à des cellules cibles (2), compris dans une population hétérogène de cellules, par :
a) la fourniture d'une population hétérogène de cellules comprenant une pluralité de populations de clones cellulaires portant un récepteur ;
b) la fourniture de cellules cibles fixées à une surface (1) ;
c) la mise en contact de la population hétérogène de cellules avec les cellules cibles ;
d) l'application d'une force (7) sur la population hétérogène de cellules à l'opposé des cellules cibles ;
e) la collecte de cellules détachées pour obtenir au moins une fraction cellulaire;
f) l'identification de clones cellulaires compris dans la ou les fraction(s) obtenue(s) à l'étape e), et la détermination du nombre de cellules représentant les clones cellulaires identifiés dans la fraction ;
g) l'attribution de scores d'avidité cellulaire aux clones cellulaires identifiés sur la base du nombre de cellules déterminé à l'étape f).

2. Procédé selon la revendication 1, dans lequel, en plus ou au lieu de la collecte des cellules détachées, les cellules qui restent fixées aux cellules cibles à la fin de la force appliquée sont collectées sous la forme d'une fraction à l'étape e).

3. Procédé selon la revendication 1 ou 2, dans lequel, parmi la population hétérogène de cellules fournie à l'étape a), est collectée une fraction de la population de départ fournie à l'étape a) qui représente une fraction supplémentaire fournie à l'étape e) ; et/ou dans lequel la population hétérogène de cellules fournie à l'étape a) est définie en fonction des populations de clones cellulaires qu'elle comprend.

4. Procédé selon l'une quelconque des revendications 1 à 3, dans lequel, en outre, une partie définie de cellules de contrôle et/ou de cellules de référence est incluse dans la population hétérogène de cellules fournie à l'étape a).

5. Procédé selon l'une quelconque des revendications 1 à 4, dans lequel le nombre de cellules représentant un clone cellulaire est déterminé par quantification des séquences d'acide nucléique représentant un clone cellulaire.

6. Procédé selon l'une quelconque des revendications 1 à 5, dans lequel, pour un clone cellulaire identifié, le pourcentage de cellules qui sont restées liées par rapport à la population de clones cellulaires initiale comprise dans la population hétérogène est calculé en tant qu'un score d'avidité cellulaire.

7. Procédé selon l'une quelconque des revendications 1 à 6, comprenant en outre le classement des clones cellulaires identifiés et de séquences de récepteur sur la base des scores d'avidité.

8. Procédé selon l'une quelconque des revendications 1 à 7, dans lequel la force appliquée est une augmentation graduelle de force, de préférence une augmentation graduelle de force linéaire.

9. Procédé selon l'une quelconque des revendications 1 à 8, dans lequel la force appliquée est une force acoustique, une force d'écoulement en cisaillement ou une force d'accélération.

10. Procédé selon l'une quelconque des revendications 1 à 9, dans lequel les cellules cibles sont fixées à une surface en verre ou en plastique, de préférence sous la forme d'une monocouche.

11. Procédé selon l'une quelconque des revendications 1 à 10, dans lequel les cellules cibles sont des cellules cancéreuses ou des cellules présentant un antigène d'un cancer.

12. Procédé selon l'une quelconque des revendications 1 à 11, dans lequel les cellules avec un récepteur sont des cellules effectrices immunitaires.

13. Procédé selon la revendication 12, dans lequel les cellules effectrices sont choisies parmi les lymphocytes T, les cellules tueuses naturelles (en anglais, NK cells), les monocytes, les macrophages et les cellules dendritiques.

14. Procédé selon l'une quelconque des revendications 1 à 13, dans lequel le récepteur est un récepteur antigénique chimérique (en anglais, CAR), un récepteur des cellules T (en anglais, TCR), un corécepteur stimulateur ou un corécepteur inhibiteur.

15. Procédé selon l'une quelconque des revendications 1 à 14, dans lequel un agent capable de moduler l'interaction entre les clones cellulaires avec un récepteur et la cellule cible est inclus dans l'étape c) et les étapes subséquentes.
